# EUROPÄISCHE PATENTSCHRIFT

(11) **EP 2 750 689 B1**
(45) Veröffentlichungstag und Bekanntmachung des Hinweises auf die Patenterteilung: **21.08.2019**
(21) Anmeldenummer: 12774981.0
(22) Anmeldetag: 21.09.2012
(51) Int. Cl.: A61K 38/10

(54) **MODIFIZIERTE APIDAECINDERIVATE ALS ANTIBIOTISCHE PEPTIDE**
MODIFIED APIDAECIN DERIVATIVES AS ANTIBIOTIC PEPTIDES
DÉRIVÉS D'APIDAECINE UTILISÉS COMME PEPTIDES ANTIBIOTIQUES

(30) Priorität: 22.09.2011 DE 102011118026
(43) Veröffentlichungstag der Anmeldung: 09.07.2014
(73) Patentinhaber: Universität Leipzig, 04109 Leipzig (DE)
(72) Erfinder: HOFFMANN, Ralf, 04463 Großpösna (DE); KNAPPE, Daniel, 04105 Leipzig (DE); HILPERT, Kai, 76356 Weingarten (DE); MIKUT, Ralf, 76227 Kalrsruhe (DE); RUDEN, Serge, 75173 Pforzheim (DE)
(74) Vertreter: CH Kilger Anwaltspartnerschaft mbB
(86) Internationale Anmeldenummer: PCT/EP2012/068620
(87) Internationale Veröffentlichungsnummer: WO 2013/041663

(56) Entgegenhaltungen:
- WO-A1-95/23513
- WO-A1-2012/175532
- DE-A1-102007 036 128
- HILPERT K ET AL: "Screening and Characterization of Surface-Tethered Cationic Peptides for Antimicrobial Activity", CHEMISTRY AND BIOLOGY, CURRENT BIOLOGY, LONDON, GB, Bd. 16, Nr. 1, 30. Januar 2009 (2009-01-30), Seiten 58-69, XP025897061, ISSN: 1074-5521, DOI: 10.1016/J.CHEMBIOL.2008.11.006 [gefunden am 2009-01-30]
- S. TAGUCHI ET AL: "Targeted Engineering of the Antibacterial Peptide Apidaecin, Based on an In Vivo Monitoring Assay System", APPLIED AND ENVIRONMENTAL MICROBIOLOGY, Bd. 75, Nr. 5, 1. März 2009 (2009-03-01), Seiten 1460-1464, XP055050602, ISSN: 0099-2240, DOI: 10.1128/AEM.02096-08
- PENG XU ET AL: "Antimicrobial Peptide Evolution in the Asiatic Honey Bee Apis cerana", PLOS ONE, Bd. 4, Nr. 1, 1. Januar 2009 (2009-01-01), Seite e4239, XP055058089, ISSN: 1932-6203, DOI: 10.1371/journal.pone.0004239

### SEQUENCE LISTING

<110> AMP-Therapeutics GmbH
<120> Modifizierte Apidaecinderivate als antibiotische Peptide
<130> B108-0007WO1
<150> DE102011118026.9
   <151> 2011-09-22
<160> 91
<170> PatentIn version 3.5
<210> 1
   <211> 18
   <212> PRT
   <213> Apis mellifera
<400> 1
   Gly Asn Asn Arg Pro Val Tyr Ile Pro Gln Pro Arg Pro Pro His Pro
   1 5 10 15
   Arg Ile
<210> 2
   <211> 18
   <212> PRT
   <213> Apis mellifera
<400> 2
   Gly Asn Asn Arg Pro Val Tyr Ile Pro Gln Pro Arg Pro Pro His Pro
   1 5 10 15
   Arg Leu
<210> 3
   <211> 16
   <212> PRT
   <213> Artificial Sequence
<220>
   <223> Artificial Peptide
<220>
   <221> misc_feature
   <222> (1)..(1)
   <223> Xaa can be any naturally occurring amino acid
<220>
   <221> misc_feature
   <222> (3)..(4)
   <223> Xaa can be any naturally occurring amino acid
<220>
   <221> misc_feature
   <222> (10)..(11)
   <223> Xaa can be any naturally occurring amino acid
<400> 3
   Xaa Asn Xaa Xaa Pro Val Tyr Ile Pro Xaa Xaa Arg Pro Pro His Pro
   1               5                   10                  15
<210> 4
   <211> 6
   <212> PRT
   <213> Artificial Sequence
<220>
   <223> Artificial Peptide
<220>
   <221> misc_feature
   <222> (1)..(1)
   <223> Xaa can be any naturally occurring amino acid
<220>
   <221> misc_feature
   <222> (3)..(5)
   <223> Xaa can be any naturally occurring amino acid
<400> 4
   Xaa Pro Xaa Xaa Xaa Pro
   1               5
<210> 5
   <211> 8
   <212> PRT
   <213> Artificial Sequence
<220>
   <223> Artificial Peptide
<220>
   <221> misc_feature
   <222> (8)..(8)
   <223> Xaa can be any naturally occurring amino acid
<400> 5
   Pro Arg Pro Pro His Pro Arg Xaa
   1               5
<210> 6
   <211> 17
   <212> PRT
   <213> Artificial Sequence
<220>
   <223> Artificial Peptide
<220>
   <221> misc_feature
   <222> (6)..(6)
   <223> Xaa can be any naturally occurring amino acid
<400> 6
   Gly Asn Asn Arg Pro Xaa Tyr Ile Pro Gln Pro Arg Pro Pro His Pro
   1               5                   10                  15
   Arg
<210> 7
   <211> 19
   <212> PRT
   <213> Drosophila melanogaster
<400> 7
   Gly Lys Pro Arg Pro Tyr Ser Pro Arg Pro Thr Ser His Pro Arg Pro
   1               5                   10                  15
   Ile Arg Val
<210> 8
   <211> 16
   <212> PRT
   <213> Myrmecia gulosa
<400> 8
   Gly Arg Pro Asn Pro Val Asn Asn Lys Pro Thr Pro Tyr Pro His Leu
   1               5                   10                  15
<210> 9
   <211> 16
   <212> PRT
   <213> Artificial Sequence
<220>
   <223> Artificial Peptide
<400> 9
   Gly Trp Asn Arg Pro Val Tyr Ile Pro Arg Pro Arg Arg Pro His Pro
   1               5                   10                  15
<210> 10
   <211> 18
   <212> PRT
   <213> Artificial Sequence
<220>
   <223> Artificial Peptide
<400> 10
   Gly Trp Asn Arg Pro Val Tyr Ile Pro Arg Pro Arg Arg Pro His Pro
   1               5                   10                  15
   Arg Leu
<210> 11
   <211> 18
   <212> PRT
   <213> Artificial Sequence
<220>
   <223> Artificial Peptide
<400> 11
   Gly Trp Asn Arg Pro Val Tyr Ile Pro Arg Pro Arg Arg Pro His Pro
   1               5                   10                  15
   Arg Ile
<210> 12
   <211> 16
   <212> PRT
   <213> Artificial Sequence
<220>
   <223> Artificial Peptide
<400> 12
   Gly Trp Asn Arg Pro Val Tyr Ile Pro Gln Pro Arg Arg Pro His Pro
   1               5                   10                  15
<210> 13
   <211> 18
   <212> PRT
   <213> Artificial Sequence
<220>
   <223> Artificial Peptide
<400> 13
   Gly Trp Asn Arg Pro Val Tyr Ile Pro Gln Pro Arg Arg Pro His Pro
   1 5 10 15
   Arg Leu
<210> 14
   <211> 18
   <212> PRT
   <213> Artificial Sequence
<220>
   <223> Artificial Peptide
<400> 14
   Gly Trp Asn Arg Pro Val Tyr Ile Pro Gln Pro Arg Arg Pro His Pro
   1 5 10 15
   Arg Ile
<210> 15
   <211> 18
   <212> PRT
   <213> Artificial Sequence
<220>
   <223> Artificial Peptide
<400> 15
   Gly Trp Asn Arg Pro Val Tyr Ile Pro Gln Pro Arg Pro Pro His Pro
   1 5 10 15
   Arg Leu
<210> 16
   <211> 18
   <212> PRT
   <213> Artificial Sequence
<220>
   <223> Artificial Peptide
<400> 16
   Gly Arg Asn Arg Pro Val Tyr Ile Pro Gln Pro Arg Pro Pro His Pro
   1 5 10 15
   Arg Leu
<210> 17
   <211> 18
   <212> PRT
   <213> Artificial Sequence
<220>
   <223> Artificial Peptide
<400> 17
   Gly Asn Asn Arg Cys Val Tyr Ile Pro Gln Pro Arg Pro Pro His Pro
   1               5                   10                  15
   Arg Leu
<210> 18
   <211> 18
   <212> PRT
   <213> Artificial Sequence
<220>
   <223> Artificial Peptide
<400> 18
   Gly Asn Asn Arg Arg Val Tyr Ile Pro Gln Pro Arg Pro Pro His Pro
   1               5                   10                  15
   Arg Leu
<210> 19
   <211> 18
   <212> PRT
   <213> Artificial Sequence
<220>
   <223> Artificial Peptide
<400> 19
   Gly Asn Asn Arg Pro Val Tyr Arg Pro Gln Pro Arg Pro Pro His Pro
   1               5                   10                  15
   Arg Leu
<210> 20
   <211> 18
   <212> PRT
   <213> Artificial Sequence
<220>
   <223> Artificial Peptide
<400> 20
   Gly Asn Asn Arg Pro Val Tyr Ile Pro Gln Pro Arg Arg Pro His Pro
   1 5 10 15
   Arg Leu
<210> 21
   <211> 18
   <212> PRT
   <213> Artificial Sequence
<220>
   <223> Artificial Peptide
<400> 21
   Gly Asn Asn Arg Pro Val Tyr Ile Pro Gln Pro Arg Pro Cys His Pro
   1 5 10 15
   Arg Leu
<210> 22
   <211> 18
   <212> PRT
   <213> Artificial Sequence
<220>
   <223> Artificial Peptide
<400> 22
   Gly Asn Asn Arg Pro Val Tyr Ile Pro Gln Pro Arg Pro Pro His Cys
   1 5 10 15
   Arg Leu
<210> 23
   <211> 18
   <212> PRT
   <213> Artificial Sequence
<220>
   <223> Artificial Peptide
<400> 23
   Gly Asn Asn Arg Pro Val Tyr Ile Pro Gln Pro Arg Pro Pro His Pro
   1 5 10 15
   Arg Arg
<210> 24
   <211> 18
   <212> PRT
   <213> Artificial Sequence
<220>
   <223> Artificial Peptide
<400> 24
   Gly Asn Asn Arg Pro Val Tyr Ile Pro Arg Pro Arg Arg Pro His Pro
   1               5                   10                  15
   Arg Leu
<210> 25
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 25
   Xaa Trp Asn Arg Pro Val Tyr Ile Pro Arg Pro Arg Arg Pro His Pro
   1               5                   10                  15
   Arg Ile
<210> 26
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 26
   Xaa Trp Asn Arg Pro Val Tyr Ile Pro Arg Pro Arg Arg Pro His Pro
   1               5                   10                  15
   Arg Leu
<210> 27
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<400> 27
   Xaa Trp Asn Arg Pro Val Tyr Ile Pro Arg Pro Arg Arg Pro His Pro
   1 5 10 15
   Xaa Ile
<210> 28
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<400> 28
   Xaa Trp Asn Arg Pro Val Tyr Ile Pro Arg Pro Arg Arg Pro His Pro
   1 5 10 15
   Xaa Leu
<210> 29
   <211> 18
   <212> PRT
   <213> Artificial Sequence
<220>
   <223> Artificial Peptide
<400> 29
   Gly Trp Asn Arg Pro Val Tyr Ile Pro Arg Pro Arg Arg Pro His Pro
   1 5 10 15
   Arg Cys
<210> 30
   <211> 18
   <212> PRT
   <213> Artificial Sequence
<220>
   <223> Artificial Peptide
<400> 30
   Gly Trp Asn Arg Pro Val Tyr Ile Pro Arg Pro Arg Arg Pro His Pro
   1 5 10 15
   Arg Cys
<210> 31
   <211> 18
   <212> PRT
   <213> Artificial Sequence
<220>
   <223> Artificial Peptide
<400> 31
   Gly Trp Asn Arg Pro Val Tyr Ile Pro Gln Pro Arg Arg Pro His Pro
   1 5 10 15
   Arg Cys
<210> 32
   <211> 18
   <212> PRT
   <213> Artificial Sequence
<220>
   <223> Artificial Peptide
<400> 32
   Gly Trp Asn Arg Pro Val Tyr Ile Pro Gln Pro Arg Arg Pro His Pro
   1 5 10 15
   Arg Cys
<210> 33
   <211> 18
   <212> PRT
   <213> Artificial Sequence
<220>
   <223> Artificial Peptide
<400> 33
   Gly Trp Asn Arg Pro Val Tyr Ile Pro Gln Pro Arg Pro Pro His Pro
   1               5                   10                  15
   Arg Cys
<210> 34
   <211> 18
   <212> PRT
   <213> Artificial Sequence
<220>
   <223> Artificial Peptide
<400> 34
   Gly Arg Asn Arg Pro Val Tyr Ile Pro Gln Pro Arg Pro Pro His Pro
   1               5                   10                  15
   Arg Cys
<210> 35
   <211> 18
   <212> PRT
   <213> Artificial Sequence
<220>
   <223> Artificial Peptide
<400> 35
   Gly Asn Asn Arg Arg Val Tyr Ile Pro Gln Pro Arg Pro Pro His Pro
   1               5                   10                  15
   Arg Cys
<210> 36
   <211> 18
   <212> PRT
   <213> Artificial Sequence
<220>
   <223> Artificial Peptide
<400> 36
   Gly Asn Asn Arg Pro Val Tyr Arg Pro Gln Pro Arg Pro Pro His Pro
   1 5 10 15
   Arg Cys
<210> 37
   <211> 18
   <212> PRT
   <213> Artificial Sequence
<220>
   <223> Artificial Peptide
<400> 37
   Gly Asn Asn Arg Pro Val Tyr Ile Pro Gln Pro Arg Arg Pro His Pro
   1 5 10 15
   Arg Cys
<210> 38
   <211> 18
   <212> PRT
   <213> Artificial Sequence
<220>
   <223> Artificial Peptide
<400> 38
   Gly Asn Asn Arg Pro Val Tyr Ile Pro Gln Pro Arg Pro Pro His Pro
   1 5 10 15
   Arg Cys
<210> 39
   <211> 18
   <212> PRT
   <213> Artificial Sequence
<220>
   <223> Artificial Peptide
<400> 39
   Gly Asn Asn Arg Pro Val Tyr Ile Pro Arg Pro Arg Arg Pro His Pro
   1 5 10 15
   Arg Cys
<210> 40
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 40
   Xaa Trp Asn Arg Pro Val Tyr Ile Pro Arg Pro Arg Arg Pro His Pro
   1               5                   10                  15
   Arg Cys
<210> 41
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 41
   Xaa Trp Asn Arg Pro Val Tyr Ile Pro Arg Pro Arg Arg Pro His Pro
   1               5                   10                  15
   Arg Cys
<210> 42
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<400> 42
   Xaa Trp Asn Arg Pro Val Tyr Ile Pro Arg Pro Arg Arg Pro His Pro
   1 5 10 15
   Xaa Cys
<210> 43
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<400> 43
   Xaa Trp Asn Arg Pro Val Tyr Ile Pro Arg Pro Arg Arg Pro His Pro
   1 5 10 15
   Xaa Cys
<210> 44
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 44
   Xaa Asn Asn Arg Pro Val Tyr Ile Pro Arg Pro Arg Pro Pro His Pro
   1 5 10 15
   Arg Arg
<210> 45
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 45
   Xaa Trp Asn Arg Pro Val Tyr Ile Pro Arg Pro Arg Pro Pro His Pro
   1               5                   10                  15
   Arg Leu
<210> 46
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 46
   Xaa Asn Asn Arg Pro Val Tyr Ile Pro Arg Pro Arg Arg Pro His Pro
   1               5                   10                  15
   Arg Leu
<210> 47
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 47
   Xaa Trp Asn Arg Pro Val Tyr Ile Pro Arg Pro Arg Arg Pro His Pro
   1               5                   10                  15
   Arg Leu
<210> 48
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 48
   Xaa Asn Asn Arg Pro Val Tyr Ile Pro Arg Pro Arg Arg Pro His Pro
   1               5                   10                  15
   Arg Leu
<210> 49
   <211> 18
   <212> PRT
   <213> Artificial Sequence
<220>
   <223> Artificial Peptide
<220>
   <221> Xaa
   <222> (1)..(1)
   <223> Xaa is Ornithine
<400> 49
   Xaa Trp Asn Arg Pro Val Tyr Ile Pro Arg Pro Arg Arg Pro His Pro
   1               5                   10                  15
   Arg Ile
<210> 50
   <211> 18
   <212> PRT
   <213> Artificial Sequence
<220>
   <223> Artificial Peptide
<220>
   <221> Xaa
   <222> (18)..(18)
   <223> Xaa is Leucin amide
<220>
   <221> Xaa
   <222> (18)..(18)
   <223> Xaa is Leucine amide
<400> 50
   Gly Trp Asn Arg Pro Val Tyr Ile Pro Arg Pro Arg Arg Pro His Pro
   1               5                   10                  15
   Arg Xaa
<210> 51
   <211> 18
   <212> PRT
   <213> Artificial Sequence
<220>
   <223> Artificial Peptide
<220>
   <221> Xaa
   <222> (18)..(18)
   <223> Xaa is Isoleucine amide
<400> 51
   Gly Trp Asn Arg Pro Val Tyr Ile Pro Arg Pro Arg Arg Pro His Pro
   1               5                   10                  15
   Arg Xaa
<210> 52
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 52
   Gly Trp Asn Arg Pro Val Tyr Ile Pro Gln Pro Arg Arg Pro His Pro
   1               5                   10                  15
   Arg Xaa
<210> 53
   <211> 18
   <212> PRT
   <213> Artificial Sequence
<220>
   <223> Artificial Peptide
<220>
   <221> Xaa
   <222> (18)..(18)
   <223> Xaa is Isoleucine amide
<400> 53
   Gly Trp Asn Arg Pro Val Tyr Ile Pro Gln Pro Arg Arg Pro His Pro
   1               5                   10                  15
   Arg Xaa
<210> 54
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 54
   Gly Trp Asn Arg Pro Val Tyr Ile Pro Gln Pro Arg Pro Pro His Pro
   1               5                   10                  15
   Arg Xaa
<210> 55
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 55
   Gly Arg Asn Arg Pro Val Tyr Ile Pro Gln Pro Arg Pro Pro His Pro
   1               5                   10                  15
   Arg Xaa
<210> 56
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 56
   Gly Asn Asn Arg Cys Val Tyr Ile Pro Gln Pro Arg Pro Pro His Pro
   1               5                   10                  15
   Arg Xaa
<210> 57
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 57
   Gly Asn Asn Arg Arg Val Tyr Ile Pro Gln Pro Arg Pro Pro His Pro
   1               5                   10                  15
   Arg Xaa
<210> 58
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 58
   Gly Asn Asn Arg Pro Val Tyr Arg Pro Gln Pro Arg Pro Pro His Pro
   1 5 10 15
   Arg Xaa
<210> 59
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 59
   Gly Asn Asn Arg Pro Val Tyr Ile Pro Gln Pro Arg Arg Pro His Pro
   1 5 10 15
   Arg Xaa
<210> 60
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 60
   Gly Asn Asn Arg Pro Val Tyr Ile Pro Gln Pro Arg Pro Cys His Pro
   1 5 10 15
   Arg Xaa
<210> 61
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 61
   Gly Asn Asn Arg Pro Val Tyr Ile Pro Gln Pro Arg Pro Pro His Cys
   1               5                   10                  15
   Arg Xaa
<210> 62
   <211> 18
   <212> PRT
   <213> Artificial Sequence
<220>
   <223> Artificial Peptide
<220>
   <221> Xaa
   <222> (18)..(18)
   <223> Xaa is Arginin amide
<400> 62
   Gly Asn Asn Arg Pro Val Tyr Ile Pro Gln Pro Arg Pro Pro His Pro
   1               5                   10                  15
   Arg Xaa
<210> 63
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 63
   Gly Trp Asn Arg Pro Val Tyr Ile Pro Gln Pro Arg Arg Pro His Pro
   1               5                   10                  15
   Arg Xaa
<210> 64
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 64
   Gly Asn Asn Arg Pro Val Tyr Ile Pro Arg Pro Arg Arg Pro His Pro
   1               5                   10                  15
   Arg Xaa
<210> 65
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (18)..(18)
   <223> Xaa is Isoleucine amide
<400> 65
   Xaa Trp Asn Arg Pro Val Tyr Ile Pro Arg Pro Arg Arg Pro His Pro
   1               5                   10                  15
   Arg Xaa
<210> 66
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (18)..(18)
   <223> Xaa is Leucine amide
<400> 66
   Xaa Trp Asn Arg Pro Val Tyr Ile Pro Arg Pro Arg Arg Pro His Pro
   1               5                   10                  15
   Arg Xaa
<210> 67
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<220>
   <221> Xaa
   <222> (18)..(18)
   <223> Xaa is Isoleucine amide
<400> 67
   Xaa Trp Asn Arg Pro Val Tyr Ile Pro Arg Pro Arg Arg Pro His Pro
   1               5                   10                  15
   Xaa Xaa
<210> 68
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<220>
   <221> Xaa
   <222> (18)..(18)
   <223> Xaa is Leucine amide
<400> 68
   Xaa Trp Asn Arg Pro Val Tyr Ile Pro Arg Pro Arg Arg Pro His Pro
   1               5                   10                  15
   Xaa Xaa
<210> 69
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 69
   Gly Trp Asn Arg Pro Val Tyr Ile Pro Arg Pro Arg Arg Pro His Pro
     1               5                   10                  15
   Arg Xaa
<210> 70
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 70
   Gly Trp Asn Arg Pro Val Tyr Ile Pro Arg Pro Arg Arg Pro His Pro
   1               5                   10                  15
   Arg Xaa
<210> 71
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 71
   Gly Trp Asn Arg Pro Val Tyr Ile Pro Gln Pro Arg Arg Pro His Pro
   1               5                   10                  15
   Arg Xaa
<210> 72
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 72
   Gly Trp Asn Arg Pro Val Tyr Ile Pro Gln Pro Arg Arg Pro His Pro
   1               5                   10                  15
   Arg Xaa
<210> 73
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 73
   Gly Trp Asn Arg Pro Val Tyr Ile Pro Gln Pro Arg Pro Pro His Pro
   1 5 10 15
   Arg Xaa
<210> 74
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 74
   Gly Arg Asn Arg Pro Val Tyr Ile Pro Gln Pro Arg Pro Pro His Pro
   1 5 10 15
   Arg Xaa
<210> 75
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 75
   Gly Asn Asn Arg Arg Val Tyr Ile Pro Gln Pro Arg Pro Pro His Pro
   1 5 10 15
   Arg Xaa
<210> 76
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 76
   Gly Asn Asn Arg Pro Val Tyr Arg Pro Gln Pro Arg Pro Pro His Pro
   1 5 10 15
   Arg Xaa
<210> 77
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 77
   Gly Asn Asn Arg Pro Val Tyr Ile Pro Gln Pro Arg Arg Pro His Pro
   1 5 10 15
   Arg Xaa
<210> 78
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 78
   Gly Asn Asn Arg Pro Val Tyr Ile Pro Gln Pro Arg Pro Pro His Pro
   1 5 10 15
   Arg Xaa
<210> 79
   <211> 18
   <212> PRT
   <213> Artificial Sequence
<220>
   <223> Artificial Peptide
<220>
   <221> Xaa
   <222> (18)..(18)
   <223> Xaa is Cysteine amide
<400> 79
   Gly Asn Asn Arg Pro Val Tyr Ile Pro Arg Pro Arg Arg Pro His Pro
   1               5                   10                  15
   Arg Xaa
<210> 80
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (18)..(18)
   <223> Xaa is Cysteine amide
<400> 80
   Xaa Trp Asn Arg Pro Val Tyr Ile Pro Arg Pro Arg Arg Pro His Pro
   1               5                   10                  15
   Arg Xaa
<210> 81
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (18)..(18)
   <223> Xaa is Cysteine amide
<400> 81
   Xaa Trp Asn Arg Pro Val Tyr Ile Pro Arg Pro Arg Arg Pro His Pro
   1 5 10 15
   Arg Xaa
<210> 82
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<220>
   <221> Xaa
   <222> (18)..(18)
   <223> Xaa is Cysteine amide
<400> 82
   Xaa Trp Asn Arg Pro Val Tyr Ile Pro Arg Pro Arg Arg Pro His Pro
   1 5 10 15
   Xaa Xaa
<210> 83
   <211> 18
   <212> PRT
   <213> Artificial Sequence
<220>
   <223> Artificial Peptide
<220>
   <221> Xaa
   <222> (1)..(1)
   <223> Xaa is Ornithine
<220>
   <221> Xaa
   <222> (17)..(17)
   <223> Xaa is Ornithine
<220>
   <221> Xaa
   <222> (18)..(18)
   <223> Xaa is Cysteine amide
<400> 83
   Xaa Trp Asn Arg Pro Val Tyr Ile Pro Arg Pro Arg Arg Pro His Pro
   1               5                   10                  15
   Xaa Xaa
<210> 84
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<400> 84
   Xaa Asn Asn Arg Pro Val Tyr Ile Pro Arg Pro Arg Pro Pro His Pro
   1               5                   10                  15
   Arg Arg
<210> 85
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<400> 85
   Xaa Trp Asn Arg Pro Val Tyr Ile Pro Arg Pro Arg Pro Pro His Pro
   1               5                   10                  15
   Arg Leu
<210> 86
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<400> 86
   Xaa Asn Asn Arg Pro Val Tyr Ile Pro Arg Pro Arg Arg Pro His Pro
   1               5                   10                  15
   Arg Leu
<210> 87
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<400> 87
   Xaa Trp Asn Arg Pro Val Tyr Ile Pro Arg Pro Arg Arg Pro His Pro
   1               5                   10                  15
   Arg Leu
<210> 88
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<220>
   <221> Xaa
   <222> (18)..(18)
   <223> Xaa is Leucine amide
<400> 88
   Xaa Asn Asn Arg Pro Val Tyr Ile Pro Arg Pro Arg Arg Pro His Pro
   1 5 10 15
   Arg Xaa
<210> 89
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<220>
   <221> Xaa
   <222> (18)..(18)
   <223> Xaa is Leucine amide
<400> 89
   Xaa Trp Asn Arg Pro Val Tyr Ile Pro Arg Pro Arg Arg Pro His Pro
   1 5 10 15
   Arg Xaa
<210> 90
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 90
   Gly Asn Asn Arg Pro Val Tyr Ile Pro Gln Pro Arg Pro Pro His Pro
   1 5 10 15
   Arg Xaa
<210> 91
   <211> 18
   <212> PRT
   <213> Artificial Sequence
<220>
   <223> Artificial Peptide
<220>
   <221> Xaa
   <222> (18)..(18)
   <223> Xaa is Leucine amide
<400> 91
   Gly Asn Asn Asp Pro Val Tyr Ile Pro Gln Pro Arg Pro Pro His Pro
   1               5                   10                  15
   Arg Xaa
<210> 92
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<220>
   <221> Xaa
   <222> (18)..(18)
   <223> Xaa is Leucine amide
<400> 92
   Xaa Asn Asn Arg Pro Val Tyr Ile Pro Arg Pro Arg Pro Pro His Pro
   1               5                   10                  15
   Arg Xaa
<210> 93
   <211> 18
   <212> PRT
   <213> Artificial Sequence
<220>
   <223> Artificial Peptide
<220>
   <221> Xaa
   <222> (1)..(1)
   <223> Xaa is alpha-N-Guanido-Ornithine
<400> 93
   Xaa Asn Asn Arg Pro Val Tyr Ile Pro Arg Pro Arg Pro Pro His Pro
   1               5                   10                  15
   Arg Leu

## Beschreibung

### EINLEITUNG

Diese Erfindung betrifft modifizierte antibiotische Peptide, insbesondere für die Verwendung in der Medizin. Weiterhin bezieht sich die Erfindung auf Zusammensetzungen und Methoden zum Abtöten von Mikroorganismen, wie Bakterien, Viren oder Pilze, und Methoden zur Behandlung mikrobieller Infektionen.

Das Auftreten ernsthafter Bakterien- und Pilzinfektionen ist ein steigendes Problem trotz bemerkenswerter Fortschritte in der Antibiotikatherapie. Jedes Jahr gibt es mehr als 40 Millionen Krankenhausaufenthalte in den Vereinigten Staaten von Amerika und mehr als 2 Millionen Patienten infizieren sich im Krankenhaus. In 50-60% dieser Fälle sind Antibiotika-resistente Bakterien involviert. Diese im Krankenhaus erworbenen Krankheiten führen zu schätzungsweise 60.000-70.000 Todesfällen in den USA und bis zu 10.000 Todesfällen in Deutschland.

Dies verdeutlicht die Notwendigkeit der weiteren Suche nach neuen Antibiotika. Induzierbare antibakterielle Peptide repräsentieren ein Forschungsfeld, in dem die heutige Biochemie, Immunologie und Wirkstoffforschung zusammentreffen. Peptidantibiotika, mit einer Größe von 13 bis zu mehr als hundert Aminosäuren, wurden aus Pflanzen, Tieren und Mikroben isoliert (Boman, H.G. 1995).

Ein einzelnes Tier besitzt ca. 6-10 antimikrobielle Peptide, wobei jedes Peptid oft ein komplett anderes Aktivitätsspektrum zeigt (Barra, D et al. 1998). Es ist bekannt, dass die große Anzahl antibakterieller Peptide, einschließlich der gut untersuchten Defensine, Cecropine und Magainine, durch einen "lytischen/ionischen" Mechanismus wirken. Als gemeinsamer Wirkmechanismus dieser "lytischen" Peptide wird ein permeabilisierender Effekt auf die bakterielle Zytoplasmamembran diskutiert. Eine kationische, amphipathische Struktur, die hydrophile Ionen-(Protonen-) Kanäle in einer Lipiddoppelschicht ausbildet, ist Grundlage dieser Aktivität. Durch das Austreten von Ionen wird das für viele grundlegende Lebensprozesse notwendige Membranpotential zerstört und so die Zelle abgetötet. Diese lytischen Peptide wirken oft in höheren Konzentrationen toxisch auf Zellmembranen von Säugetieren, was ihre Eignung als mögliche Arzneimittel einschränkt. Wird Prolin in die Sequenz der α-helikalen antimikrobiellen Peptide eingefügt, so sinkt, in Abhängigkeit von der Anzahl der Prolin-Reste, die Fähigkeit der Peptide, die Zytoplasmamembran von *E. coli* zu permeabilisieren. Bei dieser Betrachtung ist es verblüffend, dass einige der aktivsten, nativen antibakteriellen Peptide, zumindest in Bezug auf einige Gram-negative Pathogene, zu der Familie der Prolin-reichen Peptide gehören (Otvos, L. et al. 2000).

Die oben beschriebenen Nebeneffekte könnten durch antimikrobielle Peptide (AMP) überwunden werden, die ein bakterielles Protein oder andere intra- oder extrazelluläre bakterielle Komponenten spezifisch erkennen, ohne eine Kreuzreaktivität mit Säugetieranaloga zu zeigen. Dies scheint auf Prolin-reiche antimikrobielle Peptide, einschließlich Apidaecine, Drosocin und Pyrrhocoricin die ursprünglich aus Insekten isoliert wurden, zuzutreffen. Mit der enormen Variation in der Größe und in den biochemischen Eigenschaften ist es nicht überraschend, dass die Struktur-Wirkungs- und Konformations-Wirkungs-Beziehungen der Fokus der antibakteriellen Peptidforschung ist. Eine komplette Untersuchung des natürlichen, antibakteriellen Peptidrepertoires auf die biologische Stärke ist nicht nur für generelle biochemische Fragestellungen wichtig, sondern auch für die pharmazeutische Industrie von anhaltendem Interesse. Trotz der Probleme von *in vitro* Tests mit Peptid-basierenden Antibiotika, haben einige natürliche, kationische antibakterielle Peptide schon die klinische Testphase erreicht (Boman, H.G. 1995). Während einige dieser Peptide als topische (örtlich) Mittel in der frühen klinischen Testphase Wirkung zeigten, waren andere in der systemischen Therapie aktiv. Zum Beispiel hat das kationische Protein rBPI 21, das zur parentalen Behandlung von Meningococcaemia eingesetzt wird, die dritte Phase der klinischen Prüfung abgeschlossen (Boman, H.G. 1995).

Die Mitglieder der Familie der Prolin-reichen Peptide (z.B. Apidaecin, Drosocin und Pyrrhocoricin) töten Bakterien nicht durch Permeabilisierung ihrer Membran, sondern binden stereospezifisch an ein oder mehrere Zielproteine. Diese möglichen Interaktionspartner, bisher wurde das Hitzeschock Protein DnaK gut untersucht (u. a. Boman, H.G. 1995), werden durch die Prolin-reichen Peptide inhibiert und vermutlich die korrekte Proteinfaltung verhindert, was letztendlich zum Zelltod führt. Zudem scheinen Prolin-reiche Peptide, im starken Gegensatz zu AMPs mit definierter Sekundärstruktur wie Melittin oder Gramicidin, *in vitro* weder hämolytisch noch toxisch auf eukaryontische Zellen zu wirken. Entscheidenden Einfluss auf die Entwicklung neuer peptidbasierter Antibiotika hat neben der antimikrobiellen Aktivität vor allem die Stabilität in Säugetierserum (25%). So wird beispielsweise Drosocin innerhalb einer Stunde abgebaut, während Pyrrhocoricin mit Halbwertszeiten von 120 Minuten erheblich stabiler gegenüber Proteasen ist. Dabei werden vermutlich nicht nur die N- und C-Termini durch Amino- und Carboxypeptidasen abgespalten, sondern die Peptide auch durch Endoproteasen verdaut. Die dabei entstehenden Metaboliten sind teilweise stabil gegenüber weiterem Abbau, verlieren allerdings meist die antimikrobielle Aktivität (MHK-Werte ≥ 64 µg/mL).

Apidaecin ist ein Peptid, das in der Haemolymphe der Honigbienen (*Apis mellifera*) vorkommt und dort eine wichtige Rolle in der Abwehr von mikrobiellen Infektionen spielt. In Studien wurde gezeigt, dass Apidaecin hauptsächlich gegen Gram-negative Bakterien aktiv ist (Li, W. F. 2006).

US 5,300,629 A offenbart Apidaecine der allgemeinen Formel:
H₂N-G-N-N-R-P-X-Y-I-P-Q-P-R-P-P-H-P-R-Z-OH
wobei X Valin oder Isoleucin und Z Leucin oder Isoleucin ist.

WO 9523513 A offenbart Apidaecinderivate mit den Sequenzmotiven
X2-P-X3-X4-X5-P und P-R-P-P-H-P-R-X1,
wobei X1 Isoleucin oder Leucin, X2 Arginin oder Lysin, X3 Threonin, Glutamin oder Arginin, X4 Tyrosin, Glutamin oder Prolin und X5 Valin oder Alanin entspricht.

Czihal et al. 2007 erwähnt, dass die Apidaecin-Sequenz mit natürlichen und modifizierten Aminosäuren so modifiziert wurde, dass die antibakterielle Aktivität und die Proteaseresistenz erhöht wurden. Der Abstract erwähnt jedoch keine Details zu den Modifikationen, er erwähnt insbesondere keine Sequenzen und gibt keine Hinweise, welche Modifikationen erfolgreich sind.

WO 2009/013262 A1 offenbart Peptide der allgemeinen Formel:
Sub₁-X₁ N X₂ X₃ P V Y I P X₄ X₅ R P P H P-Sub₂,
wobei Sub2 Arg-Ile (RI) enthalten kann.

Gobbo et al. 2006 offenbart Apidaecin-Peptoide, in denen die Argininreste (Positionen 4, 12 und 17) durch entsprechende N-substituierte Glycine ersetzt sind. Das heißt, die Seitenkette in den Apidaecin-Peptoiden ist vom Cα zum Na-Atom verschoben. Dadurch wird die Proteaseempfindlichkeit reduziert.
In der nachfolgenden Tabelle 1 werden allgemeine Formeln und einzelne Sequenzen von bisher bekannten Apidaecin-Derivaten und verwandte Peptide in einem Alignment verglichen:

**Tabelle 1:**

| **Peptid / Dokument** | **Aminosäuresequenz*** | **SEQ ID Nr.** |
|---|---|---|
| natives Apidaecin 1a | G**N**NRPVYIPQPRP**P**HPRI | 1 |
| natives Apidaecin 1b | G**N**NRPVYIPQPR**P**PHPRL | 2 |
| WO2009013262A1 | X**N**XXPVYIPXXR**P**PHP | 3 |
| WO9523513 A | XPXXXP | 4 |
| | PR**P**PHPRX | 5 |
| US5,300,629 A | G**N**NRPXYIPQPR**P**PHPR | 6 |
| Drosocin | GKPRPYSPRPTSHPRPIRV | 7 |
| Formaecin 1 | GRPNPVNNKPTPYPHL | 8 |

| | | |
|---|---|---|
| *X ... variierte Positionen | | |

Aus Tabelle 1 geht hervor, dass in den bekannten Apidaecinderivaten insbesondere die Positionen, die den Aminosäuren Asn2, Pro5, Pro13, Pro14, His15 und Pro16 des nativen Apidaecin entsprechen, konserviert sind (dabei entsprechen die Zahlen der Position der Aminosäure in der originären Aminosäuresequenz des nativen Apidaecin).

Die Wirkung Prolin-reicher antimikrobieller Peptide ist sehr komplex, da sie die Zellmembran durchdringen und in das Zytoplasma eindringen müssen, um ein spezielles intrazelluläres bakterielles Zielmolekül zu inhibieren, ohne jedoch auf Säugetierzellen und Blutzellen toxisch zu wirken. Ein anderer wichtiger Punkt ist die Stabilität der Peptide oder Peptidderivate (auch Peptidomimetika) gegenüber dem Abbau durch Peptidasen oder Proteasen in Blut und den Bakterien. Daher hat das ideale antibiotische Peptid eine hohe antibakterielle Aktivität (kleine MHK-Werte), keine Zelltoxizität, keine hämolytische Aktivität und eine Halbwertszeit von mehreren Stunden in Blut.

Eine Aufgabe ist es, neue antibiotische Peptide zur Verfügung zu stellen, insbesondere mit verbesserter antibiotischer Wirkung und erweitertem Wirkspektrum gegen andere Bakterienstämme, insbesondere Gram-positive Bakterien wie *Staphylococcus aureus.*

### BESCHREIBUNG

Bisher wurde angenommen, dass Apidaecin und dessen Derivate nicht gegenüber Gram-positiven Bakterien wirken und es gab bisher keine Apidaecinderivate, die gegenüber Gram-positiven Bakterien wissentlich wirksam sind oder deren Aktivität war so gering, dass sie medizinisch nicht brauchbar waren. Überraschenderweise konnten die Erfinder entgegen der herrschenden Meinung solche Apidaecinderivate identifizieren, die auch gegen Gram-positive Bakterien wirkungsvoll sind.

Somit betrifft die Erfindung ein Peptid zur Verwendung als Medikament gegen eine Infektion mit Gram-positiven Bakterien bzw. gegen eine Infektion durch sowohl Gram-positiven als auch Gram-negativen Bakterien. Ebenfalls betrifft die vorliegende Erfindung ein solches Peptid oder eine pharmazeutische Zusammensetzung enthaltend ein solches Peptid, ein Peptid-Multimer umfassend mindestens zweier solcher Peptide, eine Nukleinsäure codierend für das Peptid sowie eine Wirtszelle, die die Nukleinsäure enthält. Das hier beschriebene Peptid kann auch als Antibiotikum gegen Gram-negative Bakterien eingesetzt werden. Das erfindungsgemäße Peptid wird im Folgenden beschrieben.

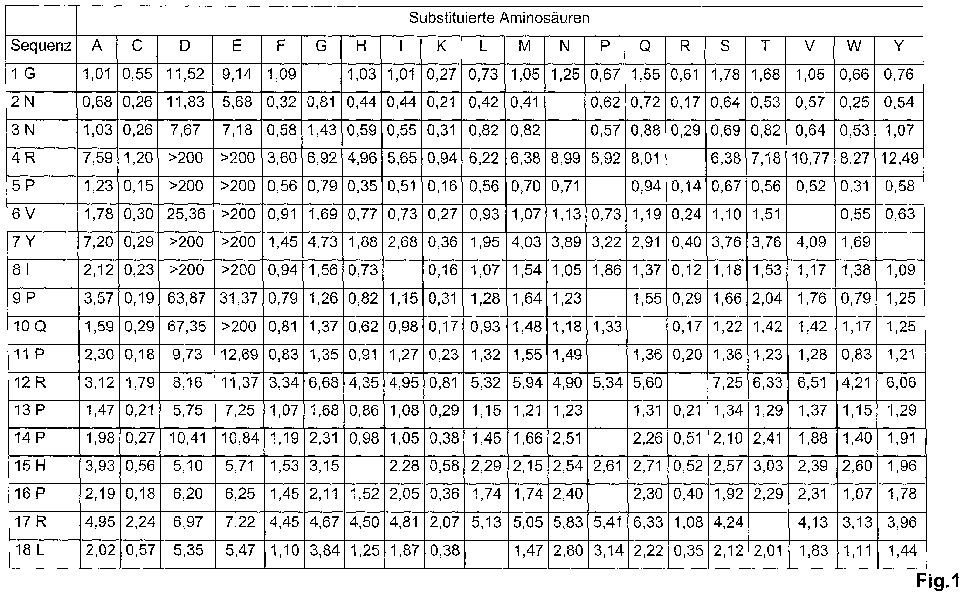

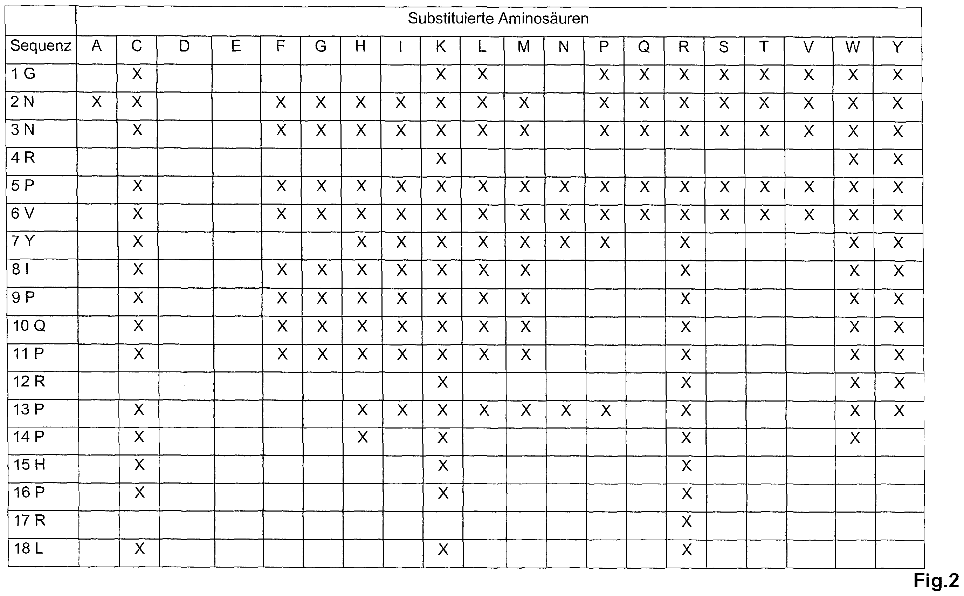

Erfindungsgemäß wird die Aufgabe durch die Peptide gemäß Figur 2 gelöst, d.h. durch diejenigen Peptide der Figur 1, deren Wert kleiner als 1 ist. Ein Wert kleiner als 1 bedeutet eine gegenüber dem nativen Apidaecin verbesserte Aktivität. Somit sind Gegenstand dieser Erfindung die in der Figur 2 dargestellten Peptide.

Des Weiteren wird die Aufgabe durch ein Peptid enthaltend eine Aminosäuresequenz gemäß der allgemeinen Formel A oder B gelöst:

X₁-N₂-X₂-X₃-P₅-V₆-Y₇-I₈-P₉-X₄-X₅-R₁₂-P₁₃-P₁₄-H₁₅-P₁₆ (FormelA)

X₁-N₂-X₂-X₃-P₅-V₆-Y₇-I₈-P₉-X₄-X₅-R₁₂-P₁₃-P₁₄-H₁₅-P₁₆-X₆-X₇ (Formel B)

Bevorzugt weist die Aminosäuresequenz gemäß Formel A oder B mindestens 80 % Aminosäuresequenzidentität zu nativem Apidaecin 1b gemäß SEQ ID Nr. 2 auf. Erfindungsgemäß gilt in Formel A oder B:
X₁ ist ausgewählt aus unpolaren, aromatischen, positiv geladenen Aminosäureresten, Aminosäureresten mit einer Thiol-Gruppe, Aminosäureresten mit einer Selenol-Gruppe, Prolin und Prolinderivaten;
N₂, X₂ und P₅ sind unabhängig voneinander ausgewählt aus neutralen und positiv geladenen Aminosäureresten;
X₃ ist ausgewählt aus positiv geladenen Aminosäureresten (vorzugsweise nicht aromatisch, insbesondere nicht Histidin), Aminosäureresten mit einer Thiol-Gruppe und Aminosäureresten mit einer Selenol-Gruppe;
V₆ ist ausgewählt aus unpolaren Aminosäureresten mit mindestens 2 C-Atomen, vorzugsweise 2 bis 8 C-Atomen, in der Seitenkette, aromatischen Aminosäureresten, positiv geladenen Aminosäureresten, Aminosäureresten mit einer Thiol-Gruppe, Aminosäureresten mit einer Selenol-Gruppe, Prolin und Prolinderivaten;
Y₇ ist ausgewählt aus Tyrosin, positiv geladenen Aminosäureresten (vorzugsweise nicht aromatisch, insbesondere nicht Histidin), Aminosäureresten mit einer Thiol-Gruppe und Aminosäureresten mit einer Selenol-Gruppe;
I₈ ist ausgewählt aus unpolaren, aromatischen Aminosäureresten mit mindestens 2 und maximal 8 C-Atomen in der Seitenkette, positiv geladenen Aminosäureresten, Aminosäureresten mit einer Thiol-Gruppe und Aminosäureresten mit einer Selenol-Gruppe;
P₉, P₁₃, P₁₄ und P₁₆ sind unabhängig voneinander ausgewählt aus positiv geladenen Aminosäureresten, Aminosäureresten mit einer Thiol-Gruppe, Aminosäureresten mit einer Selenol-Gruppe, unpolaren aromatischen Aminosäureresten, heteroaromatischen Aminosäureresten, Prolin und Prolinderivaten;
X₄ ist ausgewählt aus neutralen, positiv geladenen Aminosäureresten, Aminosäureresten mit einer Thiol-Gruppe und Aminosäureresten mit einer Selenol-Gruppe;
X₅ ist ausgewählt aus Prolin, Prolinderivaten, positiv geladenen Aminosäureresten, Aminosäureresten mit einer Thiol-Gruppe und Aminosäureresten mit einer Selenol-Gruppe;
R₁₂ ist ein positiv geladener Aminosäurerest (vorzugsweise nicht aromatisch, insbesondere nicht Histidin);
H₁₅ ist ausgewählt aus Histidin, positiv geladenen Aminosäureresten, Aminosäureresten mit einer Thiol-Gruppe und Aminosäureresten mit einer Selenol-Gruppe;
X₆ ist ausgewählt ist aus positiv geladenen Aminosäureresten, bevorzugt ist X₆ (unverändert) Arginin;
X₇ ist ausgewählt aus unpolaren Aminosäureresten (vorzugsweise mit mindestens 2 C-Atomen in der Seitenkette, bevorzugt mit 2 - 8 C-Atomen in der Seitenkette), positiv geladenen Aminosäureresten (vorzugsweise mit mindestens 2 C-Atomen in der Seitenkette, bevorzugt mit 2 - 8 C-Atomen in der Seitenkette), Aminosäureresten mit einer Thiol-Gruppe und Aminosäureresten mit einer Selenol-Gruppe.

In dem offenbarten Peptid ist zumindest eine der Positionen 2, 6, 8 bis 11, 13 bis 16 und 18 von SEQ ID No. 2 so verändert, dass auf das Peptid gemäß Formel A oder B mindestens eine der folgenden Bedingungen zutrifft:
- N₂ ist ausgewählt aus unpolaren Aminosäureresten, bevorzugt unpolaren aromatischen Aminosäureresten mit 6 bis 15, vorzugsweise 8 bis 15, C-Atomen in der Seitenkette, positiv geladenen Aminosäureresten, Aminosäureresten mit einer Thiol-Gruppe und Aminosäureresten mit einer Selenol-Gruppe, vorzugsweise ist N₂ ausgewählt aus Arginin, Ornithin, Lysin, Cystein, Seleno-Cystein, Valin, Isoleucin, Methionin, Phenylalanin und Tryptophan, besonders bevorzugt Arginin, Ornithin, Lysin, Cystein, Seleno-Cystein, Phenylalanin und Tryptophan,
- mindestens einer der Reste ausgewählt aus P₅, V₆, Y₇, I₈, P₉, P₁₃, P₁₄ und P₁₆ ist ein positiv geladener Aminosäurerest (bevorzugt Arginin, Ornithin oder Lysin), ein Aminosäurerest mit einer Thiol-Gruppe oder ein Aminosäurerest mit einer Selenol-Gruppe (bevorzugt Cystein oder Seleno-Cystein), und/oder
- H₁₅ ist ausgewählt aus Aminosäureresten mit einer Thiol-Gruppe und Aminosäureresten mit einer Selenol-Gruppe (bevorzugt Cystein oder Seleno-Cystein) und/oder
- X₇ ist ausgewählt aus positiv geladenen Aminosäureresten, Aminosäureresten mit einer Thiol-Gruppe und Aminosäureresten mit einer Selenol-Gruppe, bevorzugt ist X₇ ausgewählt aus Cystein, Lysin, Arginin und Ornithin.

Erfindungsgemäß ist das Peptid dadurch gekennzeichnet, dass zumindest eine der Positionen 2, 5 bis 11, 13 bis 16 und 18 von SEQ ID No. 2 so verändert ist, dass auf das Peptid gemäß Formel A oder B mindestens eine der folgenden Bedingungen zutrifft:
- N₂ ist Tryptophan, oder Arginin, und/oder
- I₈ ist Arginin.

In einer weiteren Ausführungsform ist das Peptid dadurch gekennzeichnet, dass zumindest eine der folgenden Positionen von SEQ ID No. 2 so verändert ist, dass auf das Peptid gemäß Formel A oder B mindestens eine der folgenden Bedingungen zutrifft:
- N₂ ist ausgewählt aus unpolaren Aminosäureresten, positiv geladenen Aminosäureresten, Aminosäureresten mit einer Thiol-Gruppe und Aminosäureresten mit einer Selenol-Gruppe, vorzugsweise ist N₂ ausgewählt aus Tryptophan, Arginin, Lysin und Cystein,
- mindestens einer der Reste ausgewählt aus P₅, V₆, Y₇, I₈, P₉, P₁₃, P₁₄, P₁₆, und X₇ ist ein positiv geladener Aminosäurerest, ein Aminosäurerest mit einer Thiol-Gruppe oder ein Aminosäurerest mit einer Selenol-Gruppe, und/oder
- H₁₅ ist ausgewählt aus Aminosäureresten mit einer Thiol-Gruppe und Aminosäureresten mit einer Selenol-Gruppe, vorzugsweise ist H₁₅ Cystein.

In einer weiteren Ausführungsform ist das Peptid dadurch gekennzeichnet, dass zumindest eine der folgenden Positionen von SEQ ID No. 2 so verändert ist, dass auf das Peptid gemäß Formel A oder B mindestens eine der folgenden Bedingungen zutrifft:
- N₂ ist Arginin oder Glutamin,
- P5 ist Cystein oder Arginin,
- mindestens einer der Reste ausgewählt aus I₈, P₁₃, P₁₄, X₇ ist Arginin, und/oder
- P₁₆ ist Cystein
und optional ist zusätzlich X₄ Arginin.

X₇ ist in bevorzugter Weise ausgewählt aus positiv geladenen Aminosäureresten, Aminosäureresten mit einer Thiol-Gruppe und Aminosäureresten mit einer Selenol-Gruppe, besonders bevorzugt ist X₇ ausgewählt aus Cystein, Lysin, Arginin und Ornithin.

In einer bestimmten Ausführungsform sind 1, 2, 3, 4 oder alle 5 Bedingungen erfüllt. Zum Beispiel können die Bedingungen von N₂ und X₄ erfüllt sein; oder die von N₂ und mindestens einer der Reste ausgewählt aus P₅, V₆, Y₇, I₈, P₉, P₁₁, P₁₃, P₁₄, P₁₆, und X₇; oder die von N₂ und H₁₅; oder die von N₂, X₄ und mindestens einer der Reste ausgewählt aus P₅, V₆, Y₇, I₈, P₉, P₁₁, P₁₃, P₁₄, P₁₆, und X₇. Es können auch die Bedingungen von X₄ und mindestens einer der Reste ausgewählt aus P₅, V₆, Y₇, I₈, P₉, P₁₁, P₁₃, P₁₄, P₁₆, und X₇ und optional H₁₅ erfüllt sein. Dem Fachmann ist bewusst, dass die Reste ausgewählt aus P₅, V₆, Y₇, I₈, P₉, P₁₁, P₁₃, P₁₄, P₁₆, und X₇ unabhängig voneinander ausgewählt werden können. Ferner ist ihm bewusst, dass die oben genannte Bedingung für 1, 2, 3, 4, 5, 6, 7, 8, 9 oder alle 10 Reste erfüllt sein kann.

Das erfindungsgemäße Peptid enthält somit keine negativ geladenen Aminosäurereste.

Für den Fall, dass sich in einem erfindungsgemäßen Peptid gemäß Formel A oder B an Position N₂ ein positiv geladener Aminosäurerest befindet, liegt dieser vorzugsweise unsubstituiert vor.

Bevorzugt ist ein erfindungsgemäßes Peptid, welches eine Aminosäuresequenz gemäß Formel A oder B enthält und in dem N₂ und/oder mindestens einer der Reste P₅, V₆, Y₇, I₈, P₉, P₁₁, P₁₃, P₁₄ und/oder P₁₆ wie oben ausgewählt ist, und in dem gilt: H₁₅ ist ausgewählt aus positiv geladenen, nicht aromatischen Aminosäureresten, Aminosäureresten mit einer Thiol-Gruppe und Aminosäureresten mit einer Selenol-Gruppe, vorzugsweise aus Cystein, Lysin, Arginin oder Ornithin.

Weiter bevorzugt ist ein erfindungsgemäßes Peptid, welches eine Aminosäuresequenz gemäß Formel A oder B enthält und in dem mindestens einer der Reste P₅, V₆, Y₇, I₈, P₉, P₁₁, P₁₃, P₁₄ und/oder P₁₆ wie oben ausgewählt ist, und in dem gilt: N₂ ist ausgewählt aus positiv geladenen, nicht aromatischen Aminosäureresten, Aminosäureresten mit einer Thiol-Gruppe und Aminosäureresten mit einer Selenol-Gruppe, vorzugsweise aus Cystein, Lysin, Arginin oder Ornithin.

Die Erfindung beruht auf einer Substitutionsanalyse von Apidaecin 1b:
GNNRPVYIPQPRPPHPRL (SEQ ID Nr. 2).

Die Substitutionsanalyse zeigte, dass die Positionen Asn2, Asn3, Pro5 im nativen Peptid besonders ungünstig besetzt und die antibiotische Aktivität durch Substitution mit beliebigen anderen, nicht negativ geladenen, Aminosäuren optimierbar ist. Auf den restlichen Positionen der Sequenz des naiven Apidaecin 1b kann die antibiotische Aktivität insbesondere durch die Substitution mit positiv geladenen Aminosäureresten (wie Lysin oder Arginin) oder durch Aminosäurereste mit einer Thiol-Gruppe (wie Cystein) verbessert werden. Interessant war die Tatsache, dass die Stellung dieser Aminosäuren innerhalb der Sequenz keine besondere Rolle zu spielen schien. Es kam allein auf ein Vorhandensein dieser Aminosäuren an (s. Figur 1). Einige der in der Substitutionsanalyse identifizierten Peptide mit verbesserter antimikrobieller Aktivität wurden weiter hinsichtlich ihrer antibakteriellen Aktivität gegen unterschiedliche Bakterienstämme, insbesondere Gram-positive Bakterien, untersucht. Dabei wurde festgestellt, dass die erfindungsgemäßen Peptide vorteilhaft auch gegen Gram-positive Bakterien, wie *S. aureus,* antibakteriell wirksam sind.

Bevorzugte erfindungsgemäße Peptide enthalten mindestens einen zusätzlichen Aminosäurerest X₆ und optional einen weiteren Aminosäurerest X₇ an dessen C-Terminus, wobei X₆ ausgewählt ist aus positiv geladenen Aminosäureresten und wobei X₇ ausgewählt ist aus positiv geladenen Aminosäureresten, Aminosäureresten mit einer Thiol-Gruppe und Aminosäureresten mit einer Selenol-Gruppe. Ein derart bevorzugtes Peptid umfasst mindestens eine Aminosäuresequenz gemäß der allgemeinen Formel B oder C:

X₁-N₂-X₂-X₃-P₅-V₆-Y₇-I₈-P₉-X₄-X₅-R₁₂-P₁₃-P₁₄-H₁₅-P₁₆-X₆ (Formel C)

Erfindungsgemäße Peptide gemäß Formel C enthalten gegenüber Formel A zusätzlich den weiteren Aminosäurerest X₆, der wie obenstehend definiert ist. Bevorzugt ist X₆ Arginin.

Bevorzugte erfindungsgemäße Peptide sind Apidaecinderivate, welche mindestens eine der folgenden Mutationen in der Aminosäuresequenz von Apidaecin 1b (SEQ ID Nr. 2) aufweisen, wobei die Zahl die Positionen innerhalb der Aminosäuresequenz gemäß SEQ ID Nr. 2 beziehen:

| | |
|---|---|
| - Asn2 → Trp, Arg, Lys oder Cys, | (Position X₁ in Formel A, B und C) |
| - Asn3 → Trp, Arg, Lys oder Cys, | (Position N₂ in Formel A, B und C) |
| - Pro5 → Cys, Lys oder Arg, | (Position P₅ in Formel A, B und C) |
| - Ile8 → Arg, Lys oder Cys, | (Position I₈ in Formel A, B und C) |
| - Pro13 → Arg, Lys oder Cys, | (Position P₁₃ in Formel A, B und C) |
| - Pro14 → Cys, Lys oder Arg, | (Position P₁₄ in Formel A, B und C) |
| - Pro16 → Arg, Cys oder Lys, | (Position P₁₆ in Formel A, B und C) |
| - Leu18 → Arg, Lys oder Cys | (Position X₇ in Formel B) |

Die verbleibenden Postionen sind unverändert oder bevorzugt wie unten und oben beschrieben ausgewählt.

Die erfindungsgemäßen Peptide weisen bevorzugt mindestens 16 Aminosäurereste, bevorzugt mindestens 18 und vorzugsweise bis zu 50 Aminosäurereste auf.

Das erfindungsgemäße Peptid enthält keine negativ geladenen Aminosäurereste. Ein negativ geladener Aminosäurerest im Sinne der Erfindung enthält eine unter physiologischen Bedingungen negativ geladene Aminosäurenseitenkette. Unter physiologischen Bedingungen wird im Sinne der Erfindung ein pH-Wert von pH 7,4, eine Temperatur von 37 °C und ein osmotischer Druck von 300 mosmol/kg verstanden. Ein positiv geladener Aminosäurerest im Sinne der Erfindung enthält eine unter physiologischen Bedingungen positiv geladene Aminosäurenseitenkette. Positiv geladene Reste sind bevorzugt nicht aromatisch und bevorzugt ausgewählt aus Arginin, Lysin, δ-Hydroxylysin, Homoarginin, 2,4-Diaminobuttersäure, β-Homoarginin, D-Arginin, Arginal (-COOH in Arginin ist ersetzt durch -CHO), 2-Amino-3-guanidinopropionsäure, 2-Amino-4-guanidinobuttersäure, Nitroarginin (bevorzugt N(G)-Nitroarginin), Nitrosoarginin (bevorzugt N(G)-Nitrosoarginin), Methylarginin (bevorzugt N-Methyl-Arginin), ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure und 3-Aminotyrosin und weniger bevorzugt sind aromatische Reste, wie Histidin, 1-Methylhistidin und 3 -Methylhistidin.

Ein neutraler Aminosäurerest enthält eine unter physiologischen Bedingungen ungeladene Aminosäureseitenkette. Neutrale Aminosäurereste sind somit unter physiologischen Bedingungen weder positiv noch negativ geladen. Der Begriff neutraler Aminosäurereste umfasst polare und unpolare Aminosäurereste.

Ein polarer Aminosäurerest weist mindestens eine polare Gruppe in der Aminosäureseitenkette auf. Diese polaren Gruppen sind unter physiologischen Bedingungen ungeladen und ausgewählt aus Hydroxyl-, Sulfhydryl-, Amin-, Amid- und Estergruppen sowie anderen Gruppen, welche die Ausbildung von Wasserstoffbrücken erlauben. Bevorzugte neutrale polare Aminosäurereste sind ausgewählt aus Asparagin, Cystein, Glutamin, Serin, Threonin, Tyrosin, Citrullin, N-Methylserin, Homoserin, allo-Threonin, 3,5-Dinitrotyrosin und β-Homoserin.

Ein unpolarer (oder auch hydrophober) Aminosäurerest weist keine polaren Gruppen auf und enthält eine unter physiologischen Bedingungen ungeladene Aminosäureseitenkette, bevorzugt mit einem Hydropathie-Index über 0, besonders bevorzugt über 3. Bevorzugte unpolare, hydrophobe Seitenketten sind ausgewählt aus H, Alkyl-, Alkylen- Alkoxy-, Alkenoxy-, Alkylsulfanyl- und Alkenylsulfanylresten mit 1 bis 10, bevorzugt 2 bis 6 C-Atomen, und Arylresten mit 5 bis 12 C-Atomen. Bevorzugte Aminosäurereste mit einer unpolaren, hydrophoben Seitenkette sind ausgewählt aus Glycin, Alanin, Leucin, Isoleucin, Valin, Methionin, Alanin, Phenylalanin, Tryptophan, *N*-Methylleucin, *tert*.-Butylglycin, Cyclohexylalanin, β-Alanin, 1-Aminocylcohexylcarbonsäure, *N*-Methylisoleucin, Norleucin, Norvalin und *N*-Methylvalin.

Aromatische Aminosäurereste weisen mindestens einen Aryl- oder Heteroarylring auf. Der Begriff umfasst polare und unpolare aromatische Aminosäurereste, wobei polar und unpolar wie oben definiert ist. Bevorzugte polare aromatische Aminosäurereste sind ausgewählt aus Arylresten mit 5 bis 12 C-Atomen, wobei diese Reste mindestens eine polare Gruppe tragen, sowie heteroaromatische Aminosäurereste. Bevorzugte heteroaromatische Aminosäurereste sind Heteroarylreste mit 3 bis 10 C-Atomen und 1 bis 4 Heteroatomen (bevorzugt N, S oder O) im Ringsystem, besonders bevorzugt Histidin. Besonders bevorzugte polare aromatische Aminosäurereste sind ausgewählt aus Tyrosin, 3,5-Dinitrotyrosin, Histidin und Histidinderivaten. Der Begriff Histidinderivat steht für einen von Histidin abgeleiteten Aminosäurenrest, der aus Histidin bevorzugt durch strukturelle Veränderung bevorzugt genau einer oder zweier funktionellen Gruppe erhalten wird. Bevorzugte Histidinderivate sind C1-C3-alkylierte (bevorzugt N-Alkyl) Histidine, insbesondere N-Methyl-Histidin. Bevorzugte unpolare aromatische Aminosäurereste sind ausgewählt aus Arylresten mit 5 bis 12 C-Atomen, wobei diese Reste keine polare Gruppen tragen. Besonders bevorzugte unpolare aromatische Aminosäurereste sind Tryptophan, Phenylalanin, Phenylglycin, Homophenylalanin, 4-tert-Butylphenylalanin, Methyl-tryptophan, Naphtylalanin, Diphenylalanin, Methylphenylalanin, Phenyl-Phenylalanin und Benzoylphenylalanin.

Aminosäurereste mit einer Thiol-Gruppe oder Selenol-Gruppe sind bevorzugt ausgewählt aus Alkyl-Alkoxy-, Alkenoxy-, Alkylsulfanyl- und Alkenylsulfanylresten mit 1 bis 10, bevorzugt 2 bis 6 C-Atomen, oder Arylresten mit 5 bis 12 C-Atomen, die mindestens eine freie (unsubstituierte) Thiol-Gruppe (-SH) oder Selenol (-SeH) Gruppe tragen. Besonders bevorzugte Aminosäurereste mit einer Thiol-Gruppe oder Selenol-Guppe sind Cystein und Seleno-Cystein.

Der Begriff Prolinderivat steht für einen von Prolin abgeleiteten Aminosäurerest, der aus Prolin bevorzugt durch strukturelle Veränderung bevorzugt genau einer oder zweier funktionellen Gruppe erhalten wird. Bevorzugte Prolinderivate sind ausgewählt aus β-Cyclohexylalanin, 3,4-cis-Methanoprolin, 3,4-Dehydroprolin, Hydroxyprolin, Mercaptoprolin, Thioprolin, Fluorprolin und Homoprolin. Der Begriff Hydroxyprolin schließt unter anderem cis-4-Hydroxyprolin, trans-4-Hydroxyprolin, cis-3-Hydroxyprolin und trans-3-Hydroxyprolin mit ein. Der Begriff Hydroxyprolinderivat steht entsprechend für einen von Hydroxyprolin abgeleiteten Aminosäurerest, der aus Hydroxyprolin bevorzugt durch strukturelle Veränderung einer funktionellen Gruppe erhalten wird. Bevorzugte Hydroxyprolinderivate sind ausgewählt aus Hydroxy-β-Cyclohexylalanin und den oben genannten Prolinderivaten, die mit einer Hydroxylgruppe substituiert sind.

In bevorzugten erfindungsgemäßen Peptiden der Formeln A, B oder C ist der Aminosäurerest X₁ bevorzugt ausgewählt ist aus Arginin, Lysin, δ-Hydroxylysin, Homoarginin, 2,4-Diaminobuttersäure, β-Homoarginin, D-Arginin, Arginal, 2-Amino-3-guanidinopropionsäure, Nitroarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, 3-Aminotyrosin, Glycin, Alanin, Valin, Isoleucin, Leucin, Methionin, N-Methylleucin, *tert*.-Butylglycin, Cyclohexylalanin, β-Alanin, 1-Aminocylcohexylcarbonsäure, N-Methylisoleucin, Norleucin, Norvalin, N-Methylvalin, Cystein, Seleno-Cystein, Phenylalanin, Tryptophan, Phenylglycin, Homophenylalanin, 4-tert-Butylphenylalanin, Methyl-tryptophan, Naphtylalanin, Diphenylalanin, Methylphenylalanin, Phenyl-Phenylalanin, Benzoylphenylalanin, Histidin, N-Methyl-Histidin, 3,5-Dinitrotyrosin, Tyrosin, Prolin, β-Cyclohexylalanin, 3,4-cis-Methanoprolin, 3,4-Dehydroprolin, Homoprolin, Mercaptoprolin, Thioprolin, Fluorprolin und Hydroxyprolin. Vorzugsweise ist X₁ ausgewählt aus unpolaren Aminosäureresten, vorzugsweise Alanin, Glycin, Phenylalanin, Methionin, Isoleucin, Valin, Leucin und Prolin, insbesondere Leucin und Prolin, und aromatischen Aminosäureresten, wie Tryptophan und Tyrosin und weniger bevorzugt Histidin, sowie besonders bevorzugt positiv geladenen Resten, insbesondere Lysin, Arginin, und Cystein. Besonders bevorzugt ist X₁ ausgewählt aus Cystein, Lysin, Arginin und Ornithin oder auch unverändert Glycin.

In bevorzugten erfindungsgemäßen Peptiden der Formeln A, B oder C sind die Aminosäurereste N₂, X₂ und P₅ ausgewählt aus allen nicht negativ geladenen Aminosäuren. Glycin ist für X₂ und Alanin für P₅ weniger bevorzugt. Die Aminosäurereste N₂, X₂ und P₅ sind bevorzugt unabhängig voneinander ausgewählt aus Arginin, Lysin, δ-Hydroxylysin, Homoarginin, β-Homoarginin, D-Arginin, Arginal, 2,4-Diaminobuttersäure, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, 3-Aminotyrosin, Asparagin, Cystein, Seleno-Cystein, Glutamin, Serin, Threonin, Citrullin, N-Methylserin, Homoserin, allo-Threonin, Tyrosin, 3,5-Dinitrotyrosin, Histidin, N-Methyl-Histidin, Phenylalanin, Tryptophan, Phenylglycin, Homophenylalanin, 4-tert-Butylphenylalanin, Methyl-tryptophan, Naphtylalanin, Diphenylalanin, Methylphenylalanin, Phenyl-Phenylalanin, Benzoylphenylalanin, β-Homoserin, Prolin, β-Cyclohexylalanin, 3,4-cis-Methanoprolin, 3,4-Dehydroprolin, Homoprolin, Mercaptoprolin, Thioprolin, Fluorprolin und Hydroxyprolin. Besonders bevorzugt ist N₂ ausgewählt aus Cystein, Tryptophan, Phenylalanin, Lysin, Arginin und Ornithin oder auch unverändert Asparagin. Besonders bevorzugt ist X₂ ausgewählt aus Cystein, Lysin, Arginin, Ornithin und Homoarginin oder auch unverändert Asparagin. Besonders bevorzugt ist P₅ ausgewählt aus Cystein, Lysin, Arginin, Ornithin, Histidin und Tryptophan oder auch unverändert Prolin.

In bevorzugten erfindungsgemäßen Peptiden der Formeln A, B oder C ist der Aminosäurerest X₃ ausgewählt aus Arginin, Lysin, δ-Hydroxylysin, Homoarginin, β-Homoarginin, D-Arginin, Arginal, 2,4-Diaminobuttersäure, β-Homoarginin, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, Cystein und Seleno-Cystein. Besonders bevorzugt ist X₃ ausgewählt aus Arginin, Lysin und Cystein, insbesondere Arginin.

In bevorzugten erfindungsgemäßen Peptiden der Formeln A, B oder C ist der Aminosäurerest V₆ ausgewählt aus Arginin, Lysin, δ-Hydroxylysin, Homoarginin, β-Homoarginin, D-Arginin, Arginal, 2,4-Diaminobuttersäure, β-Homoarginin, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, Valin, Isoleucin, Leucin, Methionin, N-Methylleucin, *tert*.-Butylglycin, Cyclohexylalanin, 1-Aminocylcohexylcarbonsäure, N-Methylisoleucin, Norleucin, Norvalin, N-Methylvalin, Phenylalanin, Phenylglycin, Homophenylalanin, 4-tert-Butylphenylalanin, Methyl-tryptophan, Naphtylalanin, Diphenylalanin, Methylphenylalanin, Phenyl-Phenylalanin, Benzoylphenylalanin, Histidin, N-Methyl-Histidin, Tryptophan, Tyrosin, Cystein, Seleno-Cystein, Prolin, β-Cyclohexylalanin, 3,4-cis-Methanoprolin, 3,4-Dehydroprolin, Homoprolin, Mercaptoprolin, Thioprolin, Fluorprolin und Hydroxyprolin. Vorzugsweise ist V₆ ausgewählt aus positiv geladenen Aminosäureresten, bevorzugt Arginin und Lysin, und Cystein und weniger bevorzugt aus aromatischen Resten, wie Histidin, N-Methyl-Histidin, Tryptophan, 3,5-Dinitrotyrosin und Tyrosin, sowie unpolaren Resten (mit mehr als zwei C-Atomen), wie Isoleucin und Prolin. Besonders bevorzugt ist V₆ ausgewählt aus Arginin, Lysin, Cystein und Tryptophan oder auch unverändert Valin.

In bevorzugten erfindungsgemäßen Peptiden der Formeln A, B oder C ist der Aminosäurerest Y₇ ausgewählt aus Tyrosin, Arginin, Lysin, δ-Hydroxylysin, Homoarginin, β-Homoarginin, D-Arginin, Arginal, 2,4-Diaminobuttersäure, β-Homoarginin, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, Cystein und Seleno-Cystein. Vorzugsweise ist Y₇ ausgewählt aus positiv geladenen Aminosäureresten, wie Arginin und Lysin, und Cystein. Besonders bevorzugt ist Y₇ ausgewählt aus Arginin, Ornithin, Lysin und Cystein oder auch unverändert Tyrosin.

In bevorzugten erfindungsgemäßen Peptiden der Formeln A, B oder C ist der Aminosäurerest I₈ ausgewählt aus Arginin, Lysin, δ-Hydroxylysin, Homoarginin, β-Homoarginin, D-Arginin, Arginal, 2,4-Diaminobuttersäure, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, 3-Aminotyrosin, Cystein, Seleno-Cystein, Valin, Isoleucin, Leucin, N-Methylleucin, *tert*.-Butylglycin, Cyclohexylalanin, 1-Aminocylcohexylcarbonsäure, N-Methylisoleucin, Norleucin, Norvalin, N-Methylvalin, Phenylalanin, Phenylglycin, Homophenylalanin, 4-tert-Butylphenylalanin, Methyl-tryptophan, Naphtylalanin, Diphenylalanin, Methylphenylalanin, Phenyl-Phenylalanin, Benzoylphenylalanin, Histidin, N-Methyl-Histidin und Tyrosin. Vorzugsweise ist I₈ ausgewählt aus positiv geladenen Aminosäureresten, wie Arginin und Lysin, und Cystein und etwas weniger bevorzugt aromatischen Resten (mit maximal 8 C-Atomen), wie Histidin, Phenylalanin und Tyrosin. Besonders bevorzugt ist I₈ ausgewählt aus Arginin, Ornithin, Lysin, Histidin und Cystein oder auch unverändert Isoleucin.

In bevorzugten erfindungsgemäßen Peptiden der Formeln A, B oder C sind die Aminosäurereste P₉, P₁₃, P₁₄ und P₁₆ unabhängig voneinander ausgewählt aus Arginin, Lysin, δ-Hydroxylysin, Homoarginin, β-Homoarginin, D-Arginin, Arginal, 2,4-Diaminobuttersäure, 2-Amino-3-guanidino-propionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, 3-Aminotyrosin, Cystein, Seleno-Cystein, Phenylalanin, Tryptophan, Phenylglycin, Homophenylalanin, 4-tert-Butylphenylalanin, Methyl-tryptophan, Naphtylalanin, Diphenylalanin, Methylphenylalanin, Phenyl-Phenylalanin, Benzoylphenylalanin, Histidin, N-Methyl-Histidin, Prolin, β-Cyclohexylalanin, 3,4-cis-Methanoprolin, 3,4-Dehydroprolin, Homoprolin, Mercaptoprolin, Thioprolin, Fluorprolin und Hydroxyprolin. Vorzugsweise sind die Aminosäurereste P₉, P₁₃, P₁₄ und P₁₆ unabhängig voneinander ausgewählt aus positiv geladenen Aminosäureresten, wie Arginin und Lysin, und Cystein und im Falle von P₉ auch weniger bevorzugt heteroaromatischen Resten, wie Histidin, und unpolaren aromatischen Aminosäureresten, Phenylalanin und Tryptophan. Besonders bevorzugt sind die Aminosäurereste P₉, P₁₃, P₁₄ und P₁₆ unabhängig voneinander ausgewählt aus Arginin, Ornithin, Lysin und Cystein oder auch unverändert Prolin.

In bevorzugten erfindungsgemäßen Peptiden der Formeln A, B oder C ist der Aminosäurerest X₄ ausgewählt aus Arginin, Lysin, δ-Hydroxylysin, Homoarginin, β-Homoarginin, D-Arginin, Arginal, 2,4-Diaminobuttersäure, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, 3-Aminotyrosin, Cystein, Seleno-Cystein, Glutamin, Citrullin, Isoleucin, Leucin, N-Methylleucin, *tert*.-Butylglycin, Cyclohexylalanin, 1-Aminocylcohexylcarbonsäure, N-Methylisoleucin, Norleucin, Norvalin, N-Methylvalin, Phenylalanin, Tryptophan, Phenylglycin, Homophenylalanin, 4-tert-Butylphenylalanin, Methyl-tryptophan, Naphtylalanin, Diphenylalanin, Methylphenylalanin, Phenyl-Phenylalanin, Benzoylphenylalanin, Histidin, N-Methyl-Histidin, 3,5-Dinitrotyrosin und Tyrosin. Vorzugsweise ist X₄ ausgewählt aus positiv geladenen Aminosäureresten, wie Arginin und Lysin, und Cystein und etwas weniger bevorzugt Isoleucin, Leucin, Histidin und Phenylalanin. Besonders bevorzugt ist X₄ ausgewählt aus Arginin, Ornithin, Lysin, Histidin und Cystein oder auch unverändert Glutamin.

In bevorzugten erfindungsgemäßen Peptiden der Formeln A, B oder C ist der Aminosäurerest X₅ ausgewählt aus Arginin, Lysin, δ-Hydroxylysin, Homoarginin, β-Homoarginin, D-Arginin, Arginal, 2,4-Diaminobuttersäure, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure, 3-Aminotyrosin, Cystein, Prolin, β-Cyclohexylalanin, 3,4-cis-Methanoprolin, 3,4-Dehydroprolin, Homoprolin, Mercaptoprolin, Thioprolin, Fluorprolin und Hydroxyprolin. Vorzugsweise ist X₅ ausgewählt aus positiv geladenen Aminosäureresten, wie Arginin und Lysin, und Cystein und etwas weniger bevorzugt unpolaren aromatischen Aminosäureresten, wie Phenylalanin und Tryptophan. Besonders bevorzugt ist X₅ ausgewählt aus Arginin, Ornithin, Lysin, Histidin und Cystein oder auch unverändert Glutamin.

In bevorzugten erfindungsgemäßen Peptiden der Formeln A, B oder C ist der Aminosäurerest R₁₂ ausgewählt aus Arginin, Lysin, δ-Hydroxylysin, Homoarginin, β-Homoarginin, D-Arginin, Arginal, 2,4-Diaminobuttersäure, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure und 3-Aminotyrosin. Besonders bevorzugt ist R₁₂ Arginin, Homoarginin, Ornithin oder Lysin.

In bevorzugten erfindungsgemäßen Peptiden der Formeln A, B oder C ist der Aminosäurerest H₁₅ ausgewählt aus Histidin, N-Methyl-Histidin, Arginin, Lysin, δ-Hydroxylysin, Homoarginin, β-Homoarginin, D-Arginin, Arginal, 2,4-Diaminobuttersäure, 2-Amino-3-guanidinopropionsäure, Nitroarginin, Nitrosoarginin, N-Methyl-Arginin, ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure und 3-Aminotyrosin und Cystein. Besonders bevorzugt ist H₁₅ Arginin, Homoarginin, Ornithin, Lysin, Cystein oder auch unverändert Histidin.

In bevorzugten erfindungsgemäßen Peptiden sind 1 bis 10, besonders bevorzugt maximal 7, ganz besonders bevorzugt maximal 5 Aminosäurereste gegenüber der nativen Aminosäuresequenz des Apidaecin 1b (SEQ ID Nr. 2) wie oben beschrieben verändert. Die übrigen Aminosäurereste entsprechen der jeweiligen Aminosäure an der entsprechenden Position im nativen Apidaecin 1b (SEQ ID Nr. 2). Bevorzugt enthält das erfindungsgemäße Peptid mindestens 3, besonders mindestens 4 positiv geladene Aminosäurenreste, besonders bevorzugt 5 bis 10 positiv geladene Aminosäurenreste. Bevorzugt enthält das Peptid mindestens 4, weiter bevorzugt mindestens 5, und bevorzugt maximal 8 Prolinreste. Weiter bevorzugt enthält das Peptid mindestens einen Cysteinrest, bevorzugt maximal drei Cysteinreste.

Bevorzugte erfindungsgemäße Peptide enthalten mindestens eine Aminosäuresequenz gemäß einer der allgemeinen Formeln 1 bis 10, wobei die Reste X₁ bis X₄ und N₂ die oben für die Formeln A, B und C genannten Bedeutungen haben und die übrigen Aminosäurereste dem IUPAC-Einbuchstabencode entsprechen:

X₁-**W**-X₂- X₂-P₅-V₆-Y₇-I₈-P₉-X₄-X₅-R₁₂-P₁₃-P₁₄-H₁₅-P₁₆,

X₁-**R**-X₂-X₃-P₅-V₆-Y₇-I₈-P₉-X₄-X₅-R₁₂-P₁₃-P₁₄-H₁₅-P₁₆,

X₁-N₂-X₂-X₃-**C**-V₆-Y₇-I₈-P₉-X₄-X₅-R₁₂-P₁₃-P₁₄-H₁₅-P₁₆,

X₁-N₂-X₂-X₃-**R**-V₆-Y₇-I₈-P₉-X₄-X₅-R₁₂-P₁₃-P₁₄-H₁₅-P₁₆,

X₁-N₂-X₂-X₃-P₅-V₆-Y₇-**R**-P₉-X₄-X₅-R₁₂-P₁₃-P₁₄-H₁₅-P₁₆,

X₁₋N₂-X₂-X₃-P₅₋V₆-Y₇₋I₈-P₉₋X₄₋X₅-R₁₂-**R**-P₁₄-H₁₅-P_{16,}

X₁₋N₂-X₂-X₃-P₅-V₆-Y₇-I₈-P₉-X₄-X₅-R₁₂-P₁₃-**C**-H₁₅-P_{16,}

X₁₋N₂-X₂-X₃-P₅-V₆-Y₇-I₈-P₉-X₄-X₅-R₁₂-P₁₃-P₁₄-H₁₅-**C**,

X₁-N₂-X₂-X₃-P₅-V₆-Y₇-I₈-P₉-X₄-X₅-R₁₂-P₁₃-P₁₄-H₁₅-P₁₆-X₆-**R**,

X₁-**W**-X₂-X₃-P₅-V₆-Y₇-I₈-P₉₋**R**-X₅-R₁₂-P₁₃-P₁₄-H₁₅-P_{16,}

X₁-**W**-X₂-X₃-P₅-V₆-Y₇-I₈-P₉-X₄-X₅-R₁₂-**R**-P₁₄-H₁₅-P₁₆,

X₁-N₂-X₂-X₃-P₅-V₆-Y₇-I₈-P₉-**R**-X₅-R₁₂-**R**-P₁₄-H₁₅-P₁₆,

X₁-**W**-X₂-X₃-P₅-V₆-Y₇-I₈-P₉-**R**-X₅-R₁₂-**R**-P₁₄-H₁₅-P_{16,}

sowie:

X₁-W-X₂-X₃-P-V-Y-I-P-X₄-R-R-P-P-H-P (Formel 1)

X₁-W-X₂-X₃-P-V-Y-I-P-X₄-P-R-P-P-H-P (Formel 2)

X₁-N₂-X₂-X₃-P-V-Y-I-P-X₄-R-R-P-P-H-P (Formel 3)

X₁-R-X₂-X₃-P-V-Y-I-P-X₄-P-R-P-P-H-P (Formel 4)

X₁-N₂-X₂-X₃-C-V-Y-I-P-X₄-P-R-P-P-H-P (Formel 5)

X₁-N₂-X₂-X₃-R-V-Y-I-P-X₄-P-R-P-P-H-P (Formel 6)

X₁-N₂-X₂-X₃-P-V-Y-R-P-X₄-P-R-P-P-H-P (Formel 7)

X₁-N₂-X₂-X₃-P-V-Y-I-P-X₄-P-R-P-C-H-P (Formel 8)

X₁-N₂-X₂-X₃-P-V-Y-I-P-X₄-P-R-P-P-H-C (Formel 9)

X₁-N₂-X₂-X₃-P-V-Y-I-P-X₄-P-R-P-P-H-P-R-R (Formel 10)

Besonders bevorzugt ist ein erfindungsgemäßes Peptid der allgemeinen Formeln A', B' oder C', wobei die Bedeutungen der einzelnen Aminosäurereste wie oben beschrieben gelten:

NT-X₁-N₂-X₂-X₃-P₅-V₆-Y₇-I₈-P₉-X₄-X₅-R₁₂-P₁₃-P₁₄-H₁₅-P₁₆-CT (Formel A')

NT-X₁-N₂-X₂-X₃-P₅-V₆-Y₇-I₈-P₉-X₄-X₅-R₁₂-P₁₃-P₁₄-H₁₅-P₁₆-X₆-CT (Formel C')

NT-X₁-N₂-X₂-X₃-P₅-V₆-Y₇-I₈-P₉-X₄-X₅-R₁₂-P₁₃-P₁₄-H₁₅-P₁₆-X₆-X₇-CT (Formel B')

Dabei ist
- NT der freie N-Terminus des Aminosäurerests X₁ oder dessen modifizierte N-terminale Aminogruppe, und
- CT die freie C-terminale Carboxylgruppe der C-terminalen Aminosäure des Peptids (-COOH), deren modifizierte C-terminale Carboxylgruppe oder ein Peptid mit bevorzugt 2 bis 6 Aminosäureresten mit einem freien oder modifizierten C-Terminus. Bevorzugt ist CT ein Dipeptid, besonders bevorzugt mit einer der folgenden Aminosäuresequenzen RF (Arg-Phe), RL (Arg-Leu), RC (Arg-Cys), RK (Arg-Lys) oder RR (Arg-Arg), mit einem freien oder modifizierten C-Terminus.

Bevorzugt ist in einem erfindungsgemäßen Peptid der allgemeinen Formeln A', B' oder C' zumindest der N-Terminus des Aminosäurerests X₁ oder der C-Terminus des Peptids modifiziert.

Weiter bevorzugte erfindungsgemäße Peptide der allgemeinen Formeln A', B' oder C' besitzen eine Aminosäuresequenz gemäß einer der allgemeinen Formeln 1' bis 10', wobei die Definitionen der einzelnen variablen Aminosäurereste X₁ bis X₄ und N₂, sowie NT und CT wie oben beschrieben gelten und die übrigen Aminosäurereste dem IUPAC-Einbuchstabencode entsprechen:

NT-X₁-W-X₂-X₃-P-V-Y-I-P-X₄-R-R-P-P-H-P-CT (Formel 1')

NT-X₁-W-X₂-X₃-P-V-Y-I-P-X₄-P-R-P-P-H-P-CT (Formel 2')

NT-X₁-X₂-X₂-X₃-P-V-Y-I-P-X₄-R-R-P-P-H-P-CT (Formel 3')

NT-X₁-R-X₂-X₃-P-V-Y-I-P-X₄-P-R-P-P-H-P-CT (Formel 4')

NT-X₁-N₂-X₂-X₃-C-V-Y-I-P-X₄-P-R-P-P-H-P-CT (Formel 5')

NT-X₁-N₂-X₂-X₃-R-V-Y-I-P-X₄-P-R-P-P-H-P-CT (Formel 6')

NT-X₁-N₂-X₂-X₃-P-V-Y-R-P-X₄-P-R-P-P-H-P-CT (Formel 7')

NT-X₁-N₂-X₂-X₃-P-V-Y-I-P-X₄-P-R-P-C-H-P-CT (Formel 8')

NT-X₁-N₂-X₂-X₃-P-V-Y-I-P-X₄-P-R-P-P-H-C-CT (Formel 9')

NT-X₁-N₂-X₂-X₃-P-V-Y-I-P-X₄-P-R-P-P-H-P-R-R-CT (Formel 10')

Unter einer "Modifikation" der N-terminalen Aminogruppe oder der C-terminalen Carboxylgruppe wird im Sinne der Erfindung verstanden, dass die Aminogruppe bzw. die Carboxylgruppe verändert sind, wie beispielsweise reduziert oder substituiert.

NT stellt somit den freien N-Terminus der Aminosäure X₁ oder eine Modifikation der N-terminalen Aminogruppe (welche die N-terminale Aminogruppe der Aminosäure X₁ durch NT ersetzt) mit der generellen Formel NR₁R₂, dar. In einer Alternative ist der N-Terminus von X₁ unmodifiziert (frei) und damit entsprechen in der generellen Formel NT = NR₁R₂ die Reste R₁ und R₂ Wasserstoff. In einer weiteren Alternative ist der N-Terminus von X₁ modifiziert, dabei ist NT = NR₁R₂, wobei die Auswahl von R₁ und R₂ unabhängig voneinander ist und die Reste R₁ und R₂ bevorzugt aus folgenden Gruppen ausgewählt werden:
(i) geradkettigen, verzweigten, zyklischen und heterozyklischen Alkylgruppen, vorzugsweise Methyl, Ethyl, n-Propyl, Isopropyl, n-Butyl, Isobutyl und Cyclohexyl;
(ii) geradkettigen, verzweigten, zyklischen und heterozyklischen Alkanoylgruppen, vorzugsweise Acetyl, Methanoyl (Formyl), Propionyl, n-Butyryl, Isobutyryl, Pentanoyl, Hexanoyl und Cyclohexanoyl;
(iii) Reportergruppen, bevorzugt Fluoreszenzfarbstoffen (vorzugsweise Fluorescein, Alexa488) und Biotin;
(iv) einen Linker zur Verbindung mit der Modifikation des C-Terminus des Peptids der allgemeinen Formel COR₃ (Definition siehe unten), zur Ausbildung eines zyklischen Peptids, vorzugsweise basierend auf Guanidin, Ethylenglycololigomeren, 2,4-Diaminobuttersäure, 2,3-Diaminopropionsäure, 2,2'-Diaminopimelinsäure, Desmosin oder Isodesmosine;
(v) Linkern zur Kopplung eines weiteren Peptids (Y₂) über eine spezifische chemische oder enzymatische Reaktion, vorzugsweise basierend auf Iod-, Brom- oder Chloralkansäuren (z.B. Iodessigsäure) oder Maleimid zur Kopplung an ein Thiolhaltiges Peptid oder auch einer anderen reaktiven Gruppe (z.B. Aminogruppe, Thiolgruppe) zur Kopplung eines zweiten Peptids oder Peptidderivats (z.B. als Aktivester, Aldehyd oder Thioester) als Träger- oder Carrierprotein,
(vi) Linkern analog zu (v), an den ein weiteres Peptid oder Peptidderivat Y₂ gekoppelt ist.

Bevorzugte N-terminale Modifikationen in einem erfindungsgemäßen Peptid sind Acetylierung, Formylierung und Guanidierung des N-Terminus. Besonders bevorzugt ist der N-Terminus eines erfindungsgemäßen Peptids guanidiert. Am meisten bevorzugt ist der N-Terminus Tetramethyl-guanidiert.

CT stellt die freie C-terminale Carboxylgruppe der C-terminalen Aminosäure (-COOH) oder eine modifizierte C-terminalen Carboxylgruppe eines erfindungsgemäßen Peptids dar. Die modifizierte C-terminalen Carboxylgruppe CT hat vorzugsweise die allgemeine Formel COR₃ (R₃ ersetzt die Hydroxylgruppe der letzten Aminosäure) oder Y₁-COR₃. Dabei ist Y₁ ist ein zusätzlicher, bevorzugt neutraler polarer oder positiv geladener, Aminosäurerest, besonders bevorzugt Leucin, Arginin oder Glutamin oder ein Peptid, mit bevorzugt zwei bis sechs Aminosäuren, insbesondere zwei bis vier Aminosäureresten, bevorzugt ein Dipeptid mit der Sequenz X₆X₇, dass bevorzugt mindestens einen neutralen polaren oder positiv geladenen Aminosäurerest enthält, der besonders bevorzugt ausgewählt ist aus Leucin und Arginin. Besonders bevorzugte Dipeptide Y₁ sind ausgewählt aus RI (Arg-Ile), RL (Arg-Leu), RV (Arg-Val), RC (Arg-Cys) und RR (Arg-Arg).

Eine bevorzugte C-terminale Modifikation eines erfindungsgemäßen Peptids ist vorzugsweise ausgewählt aus:
(i) Carboxyl (R₃ ist eine freie Hydroxylgruppe), Estern (R₃ ist eine Alkoxygruppe, vorzugsweise Methoxy, Ethoxy, Propoxy, iso-Propoxy oder Butoxy), Amiden (R₃ ist ein Amin, vorzugsweise Alkylamin, Dialkylamin, Methylamin, Ethylamin, Dimethylamin oder Cyclohexylamin) und Imiden (R₃ ist ein Amin, an welches noch ein weitere Säuregruppe, insbesondere der C-Terminus eines anderen Peptids, z. B. wie zu Y₂ genannt, eine Säuregruppe eines Polymers oder eines Trägers gebunden ist);
(ii) Linkern, die den N-Terminus des Peptids NT mit dem C-Terminus verbindet, so dass ein zyklisches Peptid gebildet wird;
(iii) C-terminalen Modifikationen der allgemeinen Formel COR₃, worin R₃ eine zusätzliche, verzweigte Aminosäure ist, um eine Dimer- oder Oligomerstruktur auszubilden, insbesondere ausgewählt aus Lysin, Hydroxylysin, Ornithin, 2,4-Diaminobuttersäure, 2,3-Diaminopropionsäure, 2,2'-Diaminopimelinsäure, Desmosin, Isodesmosin und Peptiden (vorzugsweise mit 2 - 4 Aminosäuren), die eine Kombination der vorgenannten Aminosäuren enthalten,
(iv) Linkern zur Kopplung eines weiteren Peptids (Y₂) über eine spezifische chemische oder enzymatische Reaktion, vorzugsweise basierend auf Iod-, Brom- oder Chloralkansäuren (z.B. Iodessigsäure) oder Maleimid zur Kopplung an ein Thiolhaltiges Peptid oder auch einer anderen reaktiven Gruppe (z.B. Aminogruppe, Thiolgruppe) zur Kopplung eines zweiten Peptids oder Peptidderivats (z.B. als Aktivester, Aldehyd oder Thioester) als Träger- oder Carrierprotein,
(v) Linkern analog zu (iv), an den ein weiteres Peptid oder Peptidderivat Y₂ gekoppelt ist.

Auf diesem Weg können C-terminale Peptidderivate als Ester (R₃ = Alkoxy), Amid (R₃ = Amin, z. B. -NH₂ oder Imin, z. B. -NHC₃H₇) oder Imid oder ein Peptid, das um weitere Aminosäuren verlängert wurde, die aus Pro, Ile, Arg und Val ausgewählt wurden und ebenfalls wieder am C-Terminus als Ester, Amid oder Imid modifiziert sind, erhalten werden. Weitere Peptidderivate können durch Modifikationen der N-terminalen oder C-terminalen Enden der Peptide gebildet werden. Diese Änderungen können beispielsweise eine zusätzliche Alkyl- oder Alkanoylgruppe (entweder mit einer geraden Kette oder verzweigt, zyklisch oder heterozyklisch) oder eine zusätzliche Guanidinogruppe oder ein zusätzliches Makromolekül oder ein Reporterrest sein, der entweder permanent oder durch eine Verbindung, die unter bestimmten Bedingungen spaltbar ist (wie Disulfidbrücken oder säurelabile Linker), verknüpft ist.
Bevorzugt erfolgt eine Modifikation des C-Terminus mittels Thioestersynthese und anschließender Substitution mit primären Aminen.

Die erfindungsgemäßen Peptide sind von nativem Apidaecin 1b (gemäß SEQ ID Nr. 2) abgeleitet, indem mindestens eine und vorzugsweise maximal 10 Aminosäuren der nativen Sequenz gegen eine andere Aminosäure ausgetauscht (substituiert) wurden. Ein erfindungsgemäßes Peptid weist dabei vorzugsweise eine Aminosäuresequenz auf, in der sich gegenüber SEQ ID Nr. 2 an mindestens einer der Positionen 2, 5 bis 9 und 13 bis 16 von SEQ ID Nr. 2 eine Substitution befindet. Besonders bevorzugt weist ein erfindungsgemäßes Peptid eine Aminosäuresequenz auf, in der sich gegenüber SEQ ID Nr. 2 an mindestens einer der Positionen 2, 5 und 13 bis 16 von SEQ ID Nr. 2 eine Substitution befindet, ganz besonders bevorzugt an den Positionen 2 und 13. Besonders bevorzugt umfasst das erfindungsgemäße Peptid dabei eine Aminosäuresequenz in der sich im Vergleich zu SEQ ID Nr. 2
- an Position 2 ein unpolarer Aminosäurerest, bevorzugt ein aromatischer Aminosäurerest mit 6 bis 15, vorzugsweise 8 bis 15, C-Atomen in der Seitenkette, ein positiv geladener Aminosäurerest, ein Aminosäurerest mit einer Thiol-Gruppe oder ein Aminosäurerest mit einer Selenol-Gruppe befindet, vorzugsweise ausgewählt aus Arginin, Lysin, Cystein und Tryptophan, ganz besonders bevorzugt Tryptophan, und/oder
- an mindestens einer der Positionen 5 bis 9 oder 13, 14 und 16, vorzugsweise an mindestens einer der Positionen 5, 13, 14 und 16, besonders bevorzugt an Position 13, ein positiv geladener Aminosäurerest, ein Aminosäurerest mit einer Thiol-Gruppe oder ein Aminosäurerest mit einer Selenol-Gruppe befindet, vorzugsweise ein positiv geladener Aminosäurerest, besonders bevorzugt Arginin und/oder
- an Position 15 ein positiv geladener, nicht aromatischer Aminosäurerest, ein Aminosäurerest mit einer Thiol-Gruppe oder Selenol-Gruppe oder ein Aminosäurerest mit einer Selenol-Gruppe befindet.

Besonders bevorzugt weist ein erfindungsgemäßes Peptid eine Aminosäuresequenz auf, in der sich gegenüber SEQ ID Nr. 2 an Position 2 eine Substitution mit Cystein oder Tryptophan und/oder mindestens einer der Positionen 5 und 13 bis 16 von SEQ ID Nr. 2 mindestens eine Substitution mit Cystein, Lysin oder Arginin befindet. Bevorzugt weist ein erfindungsgemäßes Peptid eine Aminosäuresequenz auf, in der sich zusätzlich gegenüber SEQ ID Nr. 2 an Position 1 Ornithin befindet.
Überraschenderweise zeigen erfindungsgemäße Peptide, insbesondere jene mit vorgenannten Substitutionen an Positionen 2, 13, 14 und/oder 18 von SEQ ID Nr. 2, (vor allem N2W, P13R, P14C und L18R) eine deutlichen Aktivitätssteigerung gegenüber *Pseudomonas aeruginosa* und sogar gegenüber dem Gram-positiven Bakterium *Staphylococcus aureus* (im Vergleich zu Apidaecin 1b). Der antimikrobielle Effekt tritt auch bei physiologischen Salzkonzentrationen auf.

In einer weiteren Ausführungsform weist das Peptid eine Aminosäuresequenz auf, in der sich gegenüber SEQ ID Nr. 2 mindestens eine der folgenden Substitution befinden:
- N₂ →: Aminosäure mit alkoholischer Hydroxygruppe im Rest; Aminosäure mit positiver oder neutraler Seitenkette mit Ausnahme von Arginin),
- X₂ →: Aminosäure mit unpolarem Rest; Aminosäure mit einer Thiol-Gruppe oder Selenol-Gruppe im Rest; aromatische Aminosäure; Aminosäure mit alkoholischer Hydroxygruppe im Rest
- P₅ →: Aliphatische Aminosäure ohne Carboxylgruppe mit Ausnahme von Alanin; aromatische Aminosäure, und/oder
- V₆ →: aromatische Aminosäure; heterozyklische Aminosäure, Aminosäure mit positivem Rest; Aminosäure mit einer Thiol-Gruppe oder Selenol-Gruppe im Rest.

Besonders bevorzugte offenbarte Peptide enthalten eine der Aminosäuresequenzen gemäß SEQ ID No. 9 bis 43 aus Tabelle 2, eine der Aminosäuresequenzen gemäß SEQ ID No. 9 bis 43 mit einem modifizierten, bevorzugt guanidierten oder acylierten, N-Terminus und/oder mit einem modifizierten, bevorzugt amidierten, C-Terminus.

**Tabelle 2:**

| **SEQ ID Nr.** | **Aminosäuresequenz** |
|---|---|
| 9 | GWNRPVYIPRPRRPHP |
| 10 | GWNRPVYIPRPRRPHPRL |
| 11 | GWNRPVYIPRPRRPHPRI |
| 12 | GWNRPVYIPQPRRPHP |
| 13 | GWNRPVYIPQPRRPHPRL |
| 14 | GWNRPVYIPQPRRPHPRI |
| 15 | GWNRPVYIPQPRPPHPRL |
| 16 | GRNRPVYIPQPRPPHPRL |
| 17 | GNNRCVYIPQPRPPHPRL |
| 18 | GNNRRVYIPQPRPPHPRL |
| 19 | GNNRPVYRPQPRPPHPRL |
| 20 | GNNRPVYIPQPRRPHPRL |
| 21 | GNNRPVYIPQPRPCHPRL |
| 22 | GNNRPVYIPQPRPPHCRL |
| 23 | GNNRPVYIPQPRPPHPRR |
| 24 | GNNRPVYIPRPRRPHPRL |
| 25 | OWNRPVYIPRPRRPHPRI |
| 26 | OWNRPVYIPRPRRPHPRL |
| 27 | OWNRPVYIPRPRRPHPOI |
| 28 | OWNRPVYIPRPRRPHPOL |
| 29 | GWNRPVYIPRPRRPHPRC |
| 30 | GWNRPVYIPRPRRPHPRC |
| 31 | GWNRPVYIPQPRRPHPRC |
| 32 | GWNRPVYIPQPRRPHPRC |
| 33 | GWNRPVYIPQPRPPHPRC |
| 34 | GRNRPVYIPQPRPPHPRC |
| 35 | GNNRRVYIPQPRPPHPRC |
| 36 | GNNRPVYRPQPRPPHPRC |
| 37 | GNNRPVYIPQPRRPHPRC |
| 38 | GNNRPVYIPQPRPPHPRC |
| 39 | GNNRPVYIPRPRRPHPRC |
| 40 | OWNRPVYIPRPRRPHPRC |
| 41 | OWNRPVYIPRPRRPHPRC |
| 42 | OWNRPVYIPRPRRPHPOC |
| 43 | OWNRPVYIPRPRRPHPOC |
| 44 | ONNRPVYIPRPRPPHPRR |
| 45 | OWNRPVYIPRPRPPHPRL |
| 46 | ONNRPVYIPRPRRPHPRL |
| 47 | OWNRPVYIPRPRRPHPRL |
| 48 | ONNRPVYIPRPRRPHPRL |
| 49 | OWNRPVYIPRPRRPHPRI |

Besonders bevorzugte offenbarte Peptide enthalten eine der Aminosäuresequenzen aus der Tabelle 2, wobei zusätzlich der C-Terminus des Peptids amidiert ist und/oder der N-Terminus guanidiert bzw. Tetramethyl-guanidiert ist (in Tabellen 3 und 6 als "gu" bezeichnet). Bevorzugte Beispiele für derart amidierte Peptide sind in Tabelle 3 aufgeführt:

**Tabelle 3:**

| **SEQ ID Nr.** | **Aminosäuresequenz** |
|---|---|
| 50 | GWNRPVYIPRPRRPHPRL-NH₂ |
| 51 | GWNRPVYIPRPRRPHPRI-NH₂ |
| 52 | GWNRPVYIPQPRRPHPRL-NH₂ |
| 53 | GWNRPVYIPQPRRPHPRI-NH₂ |
| 54 | GWNRPVYIPQPRPPHPRL-NH₂ |
| 55 | GRNRPVYIPQPRPPHPRL-NH₂ |
| 56 | GNNRCVYIPQPRPPHPRL-NH₂ |
| 57 | GNNRRVYIPQPRPPHPRL-NH₂ |
| 58 | GNNRPVYRPQPRPPHPRL-NH₂ |
| 59 | GNNRPVYIPQPRRPHPRL-NH₂ |
| 60 | GNNRPVYIPQPRPCHPRL-NH₂ |
| 61 | GNNRPVYIPQPRPPHCRL-NH₂ |
| 62 | GNNRPVYIPQPRPPHPRR-NH₂ |
| 63 | GWNRPVYIPQPRRPHPRL-NH₂ |
| 64 | GNNRPVYIPRPRRPHPRL-NH₂ |
| 65 | OWNRPVYIPRPRRPHPRI-NH₂ |
| 66 | OWNRPVYIPRPRRPHPRL-NH₂ |
| 67 | OWNRPVYIPRPRRPHPOI-NH₂ |
| 68 | OWNRPVYIPRPRRPHPOL-NH₂ |
| 69 | GWNRPVYIPRPRRPHPRC-NH₂ |
| 70 | GWNRPVYIPRPRRPHPRC-NH₂ |
| 71 | GWNRPVYIPQPRRPHPRC-NH₂ |
| 72 | GWNRPVYIPQPRRPHPRC-NH₂ |
| 73 | GWNRPVYIPQPRPPHPRC-NH₂ |
| 74 | GRNRPVYIPQPRPPHPRC-NH₂ |
| 75 | GNNRRVYIPQPRPPHPRC-NH₂ |
| 76 | GNNRPVYRPQPRPPHPRC-NH₂ |
| 77 | GNNRPVYIPQPRRPHPRC-NH₂ |
| 78 | GNNRPVYIPQPRPPHPRC-NH₂ |
| 79 | GNNRPVYIPRPRRPHPRC-NH₂ |
| 80 | OWNRPVYIPRPRRPHPRC-NH₂ |
| 81 | OWNRPVYIPRPRRPHPRC-NH₂ |
| 82 | OWNRPVYIPRPRRPHPOC-NH₂ |
| 83 | OWNRPVYIPRPRRPHPOC-NH₂ |
| 84 | gu-ONNRPVYIPRPRPPHPRR-OH |
| 85 | gu-OWNRPVYIPRPRPPHPRL-OH |
| 86 | gu-ONNRPVYIPRPRRPHPRL-OH |
| 87 | gu-OWNRPVYIPRPRRPHPRL-OH |
| 88 | gu-ONNRPVYIPRPRRPHPRL-NH₂ |
| 89 | gu-OWNRPVYIPRPRRPHPRL-NH₂ |
| 92 | gu-ONNRPVYIPRPRPPHPRL-NH₂ |
| 93 | gu-ONNRPVYIPRPRPPHPRL-OH |

Besonders bevorzugt ist der N-Terminus eines offenbarten Peptids aus Tabelle 2 guanidiert (nachfolgend NT = "Guan" oder "gu"). Bevorzugte offenbarte Peptide mit einem guanidierten N-Terminus umfassen eine Aminosäuresequenz gemäß einer der SEQ ID No. 44 bis SEQ ID No. 49, wobei bevorzugt zusätzlich der C-Terminus des Peptids amidiert ist.

Besonders bevorzugt ist das erfindungsgemäße Peptid enthaltend eine Aminosäuresequenz gemäß einer der SEQ ID Nr. 50, 52 und 54 bis 55, 58 und 63.

Alle natürlichen Aminosäuren, unnatürlichen Aminosäuren oder Aminosäurederivate (wie z.B. Iminosäuren), welche die erfindungsgemäßen Peptide oder Peptidderivate bilden, können entweder in der L- oder D-Konformation vorliegen. Wenn nicht anders spezifiziert sind die Bausteine in den Sequenzen jedoch bevorzugt in der L-Konformation.

Die Modifikationen der N- und C-Termini erlauben das Koppeln der Peptide an andere Gruppen, wie zum Beispiel andere Aminosäuresequenzen (dabei werden eventuell multimere Peptide oder Proteine geschaffen) oder andere Biomoleküle, welche die Funktion eines Carriers oder Labels haben, beispielsweise von Y₂ über NT. In einer speziellen Ausführungsform fungiert das Molekül als Träger um die bakterielle Infektion in Säugerzellen zu bekämpfen oder das antibakterielle Peptid und Peptidderivat in Bakterien zu transportieren, in die das antibakterielle Peptid nicht allein eindringen kann (z.B. Gram-positive Bakterien). Beispiele für solche Zell-penetrierenden Peptide (CPP) sind beispielsweise Penetratine, Tat-Peptide, amphipathische Modell-Peptide (model amphipathic peptides) und Transportane. Zudem kann der Ort der Infektion durch die gekoppelte Struktur (Target-Molekül) erkannt werden und dadurch die antibiotische Substanz in die Nähe der (Bakterien-)Zelle gebracht werden, um diese bekämpfen. Solche Target-Moleküle sind z.B. Moleküle, die bekanntermaßen an Lipopolysaccharid (LPS)-Moleküle binden, welche die Außenseite der Gram-negativen Bakterien bilden. Bekannte Verbindungen für diese Anwendung sind beispielsweise Ankerpeptide, wie das AcmA-Motiv aus Lactobacillus oder ein gegen Lipopolysaccharid gerichteter Antikörper. Die letztere Variante ist bevorzugt, da sie auch einen intrinsischen antibiotischen Effekt hat und deshalb zur Steigerung der Aktivität der erfindungsgemäßen Peptide genutzt werden kann.

Durch die Ankopplung einer Zell-penetrierenden Peptidsequenz, wie Penetratin, kann einerseits die Aktivität gegenüber Gram-negativen und Gram-positiven Bakterien gesteigert bzw. das Wirkungsspektrum auf andere Gram-positive und Gram-negative Bakterien erweitert werden und andererseits die antimikrobiellen Peptide in Säugerzellen eingeschleust werden, so dass auch in diesen Zellen verborgene Bakterien, Pilze oder Viren erreicht werden können. Bestandteil der Erfindung ist die Kopplung des Penetratins über eine Thioetherbrücke. Dabei wurde der C-Terminus des Penetratins um ein Cystein verlängert und an das N-terminal mit Iodessigsäure markierte antimikrobielle Peptid gekoppelt.

Der Ausdruck "Peptid", wie hier verwendet, steht für eine Sequenz von Aminosäuren, die über eine Peptidbindung verknüpft sind, wobei die Aminosäuren bevorzugt aus den zwanzig proteinogenen Aminosäuren ausgewählt sind und worin die Aminosäuren in der L-Konfiguration oder D-Konfiguration, oder im Fall von Isoleucin und Threonin auch in der D-allo-Konfiguration vorliegen können (nur Inversion eines der beiden chiralen Zentren). Umfasst sind zudem Peptidderivate, die durch Substitutionen und/oder Modifikationen von einem oder mehreren Aminosäureresten durch chemische Gruppen verändert wurden, wobei diese chemischen Gruppen andere als die natürlichen Protein-bildenden Aminosäurereste sind, wie z.B. nicht-proteinogene α-Aminosäuren, β-Aminosäuren oder Peptide mit verändertem Rückgrat. Der Begriff "verändertes Rückgrat" bedeutet, dass mindestens eine Peptidbindung chemisch modifiziert ist, d. h. ersetzt ist durch eine unter physiologischen Bedingungen nicht spaltbare Bindung, die nicht durch Endoproteasen geschnitten werden kann.

Bevorzugt ist die nicht spaltbare Bindung eine modifizierte Peptidbindung wie z. B. eine reduzierte Peptidbindung, eine alkylierte Amidbindung oder eine Thioamidbindung. Eine reduzierte Amidbindung ist eine Peptidbindung in der die Carbonylgruppe (C=O) zu einer Hydroxylgruppe (HCOH) oder einer Methylengruppe (CH₂) reduziert ist. Eine alkylierte Amidbindung ist eine entweder am Stickstoff (N-alpha) oder Kohlenstoffatom (C-alpha) alkylierte Peptidbindung. Der Alkylrest hat bevorzugt 1 bis 3 C-Atome. Ein Beispiel ist die N-Methylierung.

Außerdem umfasst der Begriff verändertes Rückgrat andere Gruppen, die geeignet sind, eine kovalente Bindung sowohl mit der COOH-Gruppe des vorangegangenen Aminosäurerestes als auch der NH₂-Gruppe des folgenden Aminosäurerestes zu bilden, und die daher nicht notwendigerweise die Peptidrückgrat-Struktur aufrecht erhalten, wie z. B. Zuckeraminosäure-Dipeptid-Isostere, Azapeptide, 6-Homopolymere, gamma-Peptide, Depsipeptide (Esterbrücken im Rückgrat), Y-Lactam-Analoga, Oligo(phenylenethylen)e, vinyloge Sulfonpeptide, poly-N-substituierte Glycine oder Oligocarbamate. Modifikationen des Rückgrats sind an Positionen bevorzugt, die anfällig für enzymatischen Abbau sind, besonders im Bereich von Argininen und Lysinen. Hier wird die Peptidbindung bevorzugt durch eine für Proteasen nicht spaltbare Bindung ersetzt. Diese nicht spaltbare Bindung ist vorzugsweise aus der Gruppe der reduzierten Amidbindungen, alkylierten Amidbindungen oder Thioamidbindungen ausgewählt.
Die erfindungsgemäßen Peptide sind bevorzugt linear. Alternativ sind die erfindungsgemäßen Peptide auch zyklisch, wobei bevorzugt die erste (N-Terminus) und die letzte Aminosäure (C-Terminus) über eine Peptidbindung oder einen Linker verknüpft sind. Umfasst sind auch Zyklisierungen zwischen einer Seitenkette (z. B. Lysin) und dem C-Terminus des Peptids, einer Seitenkette (z. B. Glutaminsäure oder Asparaginsäure) und dem N-Terminus des Peptids oder zwischen zwei Seitenketten (z. B. Lysin und Glutaminsäure oder Asparaginsäure).

Weitere Bestandteile dieser Erfindung sind die Methoden zur Herstellung der erfindungsgemäßen Peptide.

Die erfindungsgemäßen Peptide oder deren Peptidderivate können entweder synthetisch oder, wo anwendbar, rekombinant mit konventionellen Methoden hergestellt werden. Vorzugsweise werden die Peptide oder Peptidderivate dieser Erfindung konventionell mit den bekannten Synthesetechniken hergestellt, wie beispielsweise von Merrifield beschrieben. Alternativ werden die in dieser Erfindung beschriebenen Peptide durch rekombinante Techniken hergestellt, indem man ein DNA-Fragment, welches eine Nukleinsäuresequenz enthält, die für eines der oben beschriebenen Peptide kodiert, kloniert, und z. B. in einem Mikroorganismus oder einer Wirtszelle exprimiert. Die kodierenden Nukleinsäuresequenzen können synthetisch hergestellt werden oder durch seitenspezifische Mutagenese einer existierenden Nukleinsäuresequenz gewonnen werden. Die so hergestellte kodierende Sequenz kann von der RNA (oder DNA) mit entsprechend hergestellten Primern in einer Polymerasekettenreaktion (PCR) mit bekannten Techniken amplifiziert werden. Nach Reinigung, beispielsweise mittels Agarose-Gelelektrophorese, wird das PCR-Produkt in einen Vektor ligiert und letztlich die Wirtszelle mit dem entsprechenden rekombinanten Plasmid transformiert. Rekombinante Techniken sind für unterschiedliche Wirtszellen bekannt, beispielsweise *E. coli, Bacillus, Lactobacillus, Streptomyces,* Säugerzellen (z.B. CHO (Chinese hamster ovary) oder COS-1 Zellen), Hefezellen (z.B. *Saccharomyces, Schizophyllum*)*,* Insektenzellen oder virale Expressionssysteme (z.B. Baculovirus System). Nach konventioneller rekombinanter Herstellung können die Peptide dieser Erfindung aus den Wirtszellen isoliert werden, entweder mit klassischen Zellaufschlusstechniken oder vom Zellmedium mit konventionellen Methoden, z.B. Flüssigchromatographie, insbesondere der Affinitätschromatographie. Das erfindungsgemäße Peptid kann als einzelnes Peptid oder als Oligomer exprimiert werden. Dabei können die Oligomere mehrere Peptidsequenzen enthalten, die über den N- oder C-Terminus verknüpft sind, oder gar einen N- oder C-terminalen Tag enthalten, der die einfachere Reinigung der rekombinanten Peptide oder Proteinkonstrukte erlaubt. Konventionelle molekularbiologische Techniken und seitenspezifische Mutagenese können eingesetzt werden, um die Sequenz weiter zu verändern und so die gewünschten nicht-nativen Peptidsequenzen zu erhalten. Diese rekombinanten Techniken wurden bereits für viele antimikrobielle Peptide einschließlich Apidaecin (s. z. B. Maeno M et al. 1993) angewandt.
Es ist auch möglich, nicht natürlich vorkommende Aminosäuren gentechnisch in die Peptide einzubringen (Noren C et al. 1989; Ellman J et al. 1991).
Anschließend können die Peptide aus der Wirtszellkultur oder dem *in vitro-* Translationssystem isoliert werden. Dies kann mit den üblichen Techniken zur Proteinreinigung und -isolierung erreicht werden, die Stand der Technik sind. Solche Techniken können beispielsweise die Immunadsorption oder Affinitätschromatographie beinhalten. Es ist außerdem möglich, die Peptide während der Synthese mit einem Tag zu versehen (z.B. Histidin-Tag), der eine schnelle Bindung und Reinigung gestattet. Der Tag kann nachträglich enzymatisch abgespalten werden, um die aktive Peptidsequenz zu erhalten.
Falls das Peptid selbst nicht kodiert oder exprimiert werden kann, aber sehr ähnlich zu einem kodierbaren oder exprimierbaren Peptid ist, kann die Methode zunächst auf das ähnliche Peptid angewandt werden, um dieses nachträglich in einem oder mehreren Schritten chemisch oder enzymatisch in das gewünschte Peptid oder Peptidomimetika zu überführen.

Die Erfindung umfasst auch Nukleinsäuren die für die erfindungsgemäßen Peptide codieren sowie, vorzugsweise nicht-menschliche, Wirtszellen, welche eine erfindungsgemäße Nukleinsäure enthalten. Die Wirtszellen sind bevorzugt wie oben beschrieben ausgewählt und umfassen keine humanen embryonalen Stammzellen.

Die erfindungsgemäßen Peptide können einzeln, in Kombination, als Multimere oder als verzweigte Multimere eingesetzt werden. Sinnvolle Kombinationen der erfindungsgemäßen Peptide umfassen Dendrimere und Konkatamere, in denen die erfindungsgemäßen Peptide seriell miteinander oder über Spacer miteinander verknüpft sind, z. B. in der Form eines Peptiddimers oder eines Peptidtrimers usw., indem die einzelnen Peptide aneinander gereiht sind. Dieses Multimer kann aus Peptiden oder Peptidderivaten mit identischen Sequenzen oder verschiedenen Sequenzen gemäß Formel A oder B zusammengesetzt sein.

Die modifizierten Peptide können zusätzlich an ein biokompatibles Protein gekoppelt werden, beispielsweise humanes Serumalbumin, humanisierte Antikörper, Liposomen, Mizellen, synthetische Polymere, Nanopartikel und Phagen. Alternativ können Multimere, in denen die erfindungsgemäßen Peptide oder Peptidderivate individuell kombiniert sind, in der Form von Dendrimeren oder Clustern hergestellt werden, wobei drei oder mehr Peptide an ein Zentrum gebunden sind.
In einer Ausführungsform können mehrere Peptide als multimere Konstrukte oder Anordnung hergestellt werden. So können beispielsweise optional Aminosäuren (z.B. Gly-Ser-) oder andere Spacer (Linkerpeptide) basierend auf Aminosäuren oder anderen chemischen Verbindungen an den N- oder C-Terminus angehängt werden, um zwei oder mehr Peptide untereinander zu verknüpfen oder an einen Träger zu koppeln. Diese Anordnung kann die Form von einem oder mehreren der oben beschriebenen synthetischen Peptide gekoppelt an ein Trägerprotein annehmen. Alternativ enthält eine Anordnung mehrere Peptide, jedes als multiples antigenes Peptid exprimiert, optional an ein Trägerprotein gekoppelt. In einer weiteren Variante sind die ausgewählten Peptide sequentiell verknüpft und werden als rekombinantes Protein oder Polypeptid exprimiert. In einer Ausführungsform werden mehrere Peptide sequentiell, mit oder ohne Aminosäuren als Spacer (Linkerpeptid) dazwischen, verknüpft, um ein größeres rekombinantes Protein zu erhalten. Alternativ kann das rekombinante Protein an ein Trägerprotein fusioniert werden.
In einer anderen Ausführungsform enthalten die multimeren Konstrukte mindestens zwei Peptide, wobei ein Peptid über eine beliebige Aminosäure an die anderen Peptide gekoppelt wird. Eine beliebige Anzahl weiterer Peptide können an beliebige weitere Aminosäuren dieser Peptide angehängt werden. In einer weiteren Ausführungsform einer multimeren Anordnung, welches mindestens zwei Peptide enthält, ist das zweite oder sind die weiteren Peptide an ein verzweigtes Gerüst der anderen Peptide der Grundstruktur gekoppelt. Alternativ ist jedes weitere Peptid kovalent über die Gruppe NT oder CT an ein anderes Peptid der Anordnung verknüpft.
In einer anderen Ausführungsform eines multimeren Konstrukts oder einer Anordnung mit mindestens zwei Peptiden, sind mindestens eins oder mehrere Peptide an einen Träger gebunden. In einer anderen Ausführungsform sind ein oder mehrere der genannten Peptide ein synthetisches Peptid, das an ein Trägerprotein fusioniert ist. Weiterhin gibt es die Alternative mehrere der oben beschriebenen Peptide mit oder ohne flankierende Sequenzen sequentiell zu einem linearen Polypeptid zu kombinieren. Die Peptide oder das Polypeptid sind entweder an den gleichen Träger gekoppelt oder unterschiedliche Peptide können individuell als Peptide an eins oder unterschiedliche immunologisch inerte Trägerproteine gekoppelt werden.
Geeignete Träger können die Stabilität, die Darreichung oder die Produktion verbessern, oder das Aktivitätsspektrum der Peptide verändern. Beispiele für Träger sind humanes Albumin, Polyethylenglykol oder andere Biopolymere bzw. andere natürlich oder nicht-natürlich vorkommende Polymere. In einer Ausführungsform, ist die Hauptkomponente vorzugsweise ein Protein oder anderes Molekül, das die Peptidstabilität erhöhen kann. Eine erfahrene Person kann einfach eine geeignete Kopplungseinheit auswählen.
In noch einer anderen Ausführungsform sind die Peptide in der Form eines multiplen Antigenpeptides (multiple antigenic peptide; MAP) angeordnet. Dieses System nutzt eine zentrale Einheit aus Lysinresten, an die mehrere Kopien des gleichen erfindungsgemäßen Peptids synthetisiert werden. Jedes MAP enthält mehrere Kopien eines oder mehrerer der erfindungsgemäßen Peptide. Eine Ausführungsart eines MAP enthält mindestens drei, vorzugsweise aber vier oder mehr Peptide. Ein Fachmann kann einfach eine beliebige Anzahl multimerer Verbindungen gemäß den in obiger Formel identifizierten Peptiden herstellen. Alle derartigen multimeren Arrangements und Konstrukte sollen Bestandteil dieser Erfindung sein. Weitere Kombinationen in der Form von Multimeren können an der Oberfläche von Partikeln hergestellt werden, wobei die Peptide oder Peptidderivate an deren Oberfläche präsentiert werden. Der Partikel kann dann als Träger eines Peptids oder Peptidderivates fungieren und kann gleichzeitig als detektierbarer Marker wirken. Multimere können beispielsweise durch N-terminale Biotinylierung des N-terminalen Endes der Peptid- oder Peptidderivate-Ketten und anschließende Komplexbildung mit Streptavidin erhalten werden. Da Streptavidin vier Biotinmoleküle oder -konjugate mit hoher Affinität binden kann, werden mit dieser Methode sehr stabile tetramere Peptidkomplexe erhalten. Multimere können aus identischen oder unterschiedlichen erfindungsgemäßen Peptiden oder Peptidderivaten hergestellt werden. Vorzugsweise enthalten die erfindungsgemäßen Multimere zwei oder mehr Peptid oder Peptidderivaten, in welcher jede Komponente einen gewissen Anteil zur bioziden Aktivität beiträgt (Zielerkennung, antimikrobielle Aktivität, Reinigung).

Ein anderer Gegenstand dieser Erfindung ist der Einsatz der hier beschriebenen Peptide oder Peptidderivate in der Medizin oder Pharmazie, z.B. zur Therapie mit einem Antibiotikum oder in einer Zusammensetzung mit antimikrobieller (insbesondere bakteriozider) Aktivität. Bevorzugterweise wird das Peptid medizinisch als Antibiotikum gegen Gram-positive Bakterien eingesetzt.
Gegenstand der Erfindung sind auch die erfindungsgemäßen Peptide zur Verwendung in der Medizin, als Antibiotikum, in einem Desinfektions- oder Reinigungsmittel, als Konservierungsmittel oder in einem Verpackungsmaterial. Besonders eigen sich die erfindungsgemäß modifizierten Peptid zur Behandlung von mikrobiellen, bakteriellen oder Pilz-Infektionen. Gegenstand der Erfindung ist auch die Verwendung der erfindungsgemäßen Peptide zur Herstellung eines Arzneimittels, insbesondere eines Antibiotikums, insbesondere zur Behandlung von mikrobiellen Infektionen, z.B. durch Bakterien, Viren und Pilze.

Gegenstand der Erfindung ist auch die Verwendung der erfindungsgemäßen Peptide in der pharmazeutischen Forschung oder in einem Screeningverfahren, vorzugsweise in einem Screeningverfahren zur Identifikation von Substanzen, die eine antimikrobielle, bakterizide oder antimykotische Wirkung aufweisen.
Ein geeignetes derartiges Screeningverfahren zur Identifizierung einer Substanz, die voraussichtlich eine antimikrobielle, bakterizide oder antimykotische Wirkung aufweist, umfasst:
(i) die Durchführung eines kompetitiven Assays mit:
   (a) einem Mikroorganismus der gegenüber einem erfindungsgemäßen Peptid empfindlich ist;
   (b) einem erfindungsgemäßen Peptid und
   (c) mindestens einer zu testenden Substanz (Testsubstanz)
   durch in Kontakt bringen von (a) mit (b) und (c); sowie
(ii) die Auswahl einer Testsubstanz, welche kompetitiv die Bindung des Peptids an den Mikroorganismen verdrängt.

Ein weiterer Gegenstand dieser Erfindung sind pharmazeutische Zusammensetzungen, die ein oder mehrere erfindungsgemäße Peptide oder deren multimere Konstrukte unabhängig von der Anwesenheit anderer pharmazeutisch aktiver Verbindungen enthalten.

Bestandteil dieser Erfindung ist auch die Verwendung der erfindungsgemäßen Peptide als Pharmazeutikum und/oder zur Herstellung eines Wirkstoffs, der als Antibiotikum eingesetzt werden kann.
Die erfindungsgemäßen Peptide können auch einzeln in pharmazeutischen Produkten eingesetzt werden. Alternativ können ein oder mehrere modifizierte Peptide, wie oben beschrieben, an eine andere Verbindung fusioniert oder konjugiert werden, um die Pharmakokinetik oder Bioverfügbarkeit zu steigern, ohne dass eine Immunantwort ausgelöst wird. Eine beliebige Anzahl einzelner Peptide oder multimerer Konstrukte können miteinander gemischt werden, um eine einzelne Zusammensetzung herzustellen.
Eine erfindungsgemäße pharmazeutische Zusammensetzung enthält eine therapeutisch wirksame Menge eines oder mehrerer erfindungsgemäßer Peptide oder deren multimerer Konstrukte. Einmal zusammengestellt, kann die erfindungsgemäße pharmazeutische Zusammensetzung dem Subjekt direkt verabreicht werden, um mikrobielle (insbesondere bakterielle) Infektionen zu behandeln. Dazu wird dem zu behandelnden Subjekt eine therapeutisch wirksame Menge einer erfindungsgemäßen Zusammensetzung verabreicht.

Die erfindungsgemäßen pharmazeutischen Zusammensetzungen sind dazu bestimmt, Infektionen eines mit Bakterien oder Pilzen infizierten Säugetiers einschließlich des Menschen zu behandeln. Mindestens ein oder alternativ auch mehrere erfindungsgemäße Peptide oder deren multimere Konstrukte können zu einer antimikrobiell (insbesondere antibakteriell oder fungizid) wirksamen Zusammensetzung mit einem pharmakologisch vertretbaren Träger oder anderen Komponenten gemischt werden. Für den Gebrauch einer derartigen Zusammensetzung wird das ausgewählte Peptid vorzugsweise synthetisch oder auch rekombinant, wie oben beschrieben, hergestellt.
Die direkte Verabreichung der erfindungsgemäßen pharmazeutischen Zusammensetzung erfolgt lokal oder systemisch, bevorzugt oral, parenteral, intraperitoneal, intravenös, intramuskulär, pulmonal oder interstitiell ins Gewebe.
Die erfindungsgemäße pharmazeutische Zusammensetzung kann weiter geeignete und pharmazeutisch akzeptable Träger, Streckmittel, Puffer oder Lösungsmittel enthalten und die Form einer Kapsel, Tablette, Pastille, Dragee, Pille, Tropfen, Zäpfchen, Puder, Spray, Impfstoff, Salbe, Paste, Creme, Inhalat, Pflaster, Aerosol oder ähnlichem aufweisen. Als pharmazeutisch akzeptable Trägerstoffe können Lösungsmittel, Streckmittel oder andere flüssige Bindemittel wie Dispersions- oder Suspensionshilfsmittel, oberflächenaktive Agenzien, isotonische Wirkstoffe, Dickungsmittel oder Emulgatoren, Konservierungsmittel, Umhüllungsmittel (encapsulating agent), feste Bindestoffe oder Gleitmittel eingesetzt werden, je nachdem was für die jeweilige Dosierung am Besten geeignet ist und zugleich mit dem Peptid, Peptidderivat oder -Konjugat kompatibel ist.
Die erfindungsgemäße pharmazeutische Zusammensetzung enthält daher vorzugsweise einen pharmazeutisch akzeptablen Träger. Der Begriff "pharmazeutisch akzeptabler Träger" umfasst dabei auch einen Träger zur Verabreichung der therapeutischen Zusammensetzung, wie beispielsweise Antikörper oder Polypeptide, Gene oder andere therapeutische Mittel. Der Begriff bezieht sich auf einen beliebigen pharmazeutischen Träger, der selbst nicht die Produktion von Antikörpern auslöst, die für das Individuum, dem die Rezeptur verabreicht wurde, gefährlich sein könnten, und der keine unangemessene Giftigkeit besitzen. Geeignete "pharmazeutisch akzeptable Träger" können große, langsam abbaubare Makromoleküle, wie beispielsweise Proteine, Polysaccharide, Polylactonsäuren, Polyglykolsäuren, polymere Aminosäuren, Aminosäure-Kopolymere und inaktivierte Virusbestandteile sein. Solche Träger sind dem Fachmann wohlbekannt.
Salze der erfindungsgemäßen Peptide werden mit bekannten Methoden hergestellt, was typischerweise bedeutet, dass die erfindungsgemäßen Peptide, deren Peptid-Konjugate oder Konjugate mit einer pharmazeutisch akzeptablen Säure zu einem sauren Salz oder mit einer pharmazeutisch akzeptablen Base zu einem basischen Salz gemischt werden. Ob eine Säure oder eine Base pharmazeutisch akzeptabel sind, kann leicht von einem Fachmann in Kenntnis der Anwendung und der Rezeptur festgelegt werden. So sind beispielsweise nicht alle Säuren und Basen, die für *ex vivo* Anwendungen akzeptabel sind, auch auf therapeutische Rezepturen übertragbar. Abhängig von der jeweiligen Anwendung können pharmazeutisch akzeptable Säuren sowohl organischer als auch anorganischer Natur sein, z.B. Ameisensäure, Essigsäure, Propionsäure, Milchsäure, Glykolsäure, Oxalsäure, Brenztraubensäure, Bernsteinsäure, Maleinsäure, Malonsäure, Zimtsäure, Schwefelsäure, Salzsäure, Bromwasserstoffsäure, Salpetersäure, Perchlorsäure, Phosphorsäure und Thiocyansäure, die mit den freien Aminogruppen von Peptiden und funktionell äquivalenten Verbindungen Ammoniumsalze ausbilden. Pharmazeutisch akzeptable Basen, die mit freien Carbonsäuregruppen der Peptide und funktionell äquivalenten Verbindungen Carboxylate ausbilden, beinhalten Ethylamin, Methylamin, Dimethylamin, Triethylamin, Isopropylamin, Diisopropylamin und andere Mono-, Di- und Trialkylamine sowie Arylamine. Ferner sind pharmazeutisch akzeptable Lösungsmittel eingeschlossen.
Pharmazeutisch akzeptable Salze können in erfindungsgemäßen pharmazeutischen Zusammensetzungen zur Anwendung kommen, wie z. B. Salze von Mineralsäuren, wie Hydrochloride, Hydrobromide, Phosphate, Sulfate und vergleichbare; aber auch Salze organischer Säuren, wie Acetate, Propionate, Malonate, Benzoate und vergleichbare.
Pharmazeutisch akzeptable Träger in den erfindungsgemäßen pharmazeutischen Zusammensetzungen umfassen weiterhin Flüssigkeiten, beispielsweise Wasser, Salzwasser, Glycerol und Ethanol. Zusätzlich können erfindungsgemäße pharmazeutische Zusammensetzungen Hilfsstoffe enthalten, wie Befeuchtungsmittel oder Emulgatoren, pH puffernde Substanzen und ähnliche Verbindungen. Typischerweise werden die erfindungsgemäßen pharmazeutischen Zusammensetzungen entweder in flüssiger Form oder als Suspension zur Injektion zubereitet, feste Formen zum Auflösen oder Suspendieren in Trägerflüssigkeiten vor der Injektion sind ebenfalls möglich. Auch Liposomen sind von der Definition eines "pharmazeutisch akzeptablen Trägers" umfasst.
Zur therapeutischen Behandlung können erfindungsgemäße Peptide oder deren Peptid-Konjugate, wie oben beschrieben, produziert und einem Subjekt, das diese benötigt, verabreicht werden. Das Peptid oder Peptid-Konjugat kann einem Subjekt in beliebiger, geeigneter Form verabreicht werden, vorzugsweise als pharmazeutische Zusammensetzung, die der Darreichungsform angepasst ist und in einer für die angestrebte Behandlung angemessenen Dosierung vorliegt.
Die pharmazeutischen Zusammensetzungen dieser Erfindung können weitere aktive Verbindungen enthalten, beispielsweise konventionelle Antibiotika (z.B. Vancomycin, Streptomycin, Tetracyclin, Penicillin) oder andere antimikrobiell aktive Verbindungen, wie Fungizide, z.B. Intraconazol oder Myconazol. Auch andere Verbindungen, die mit der Infektion einhergehende Symptome wie Fieber (Salizylsäure) oder Hautausschlag lindern, können zugesetzt werden.

Neben der therapeutischen Verwendung zur Behandlung von Infektionen, ist es weiter möglich, die erfindungsgemäßen Peptide oder Peptidderivate in Desinfektions- oder Reinigungsmitteln (z. B. einer bakterioziden Zusammensetzung) einzusetzen, die zur Desinfektion oder Reinigung von Oberflächen oder Gegenständen verwendet werden können, insbesondere zur Vermeidung oder Entfernung von Biofilmen. Ein anderes Anwendungsgebiet sind Verpackungen, wo erfindungsgemäße Peptide an Verpackungsmaterial gebunden oder darin eingebunden werden können, oder als Konservierungsmittel für andere Materialien, die leicht durch Mikroorganismen abgebaut werden können. Die erfindungsgemäßen Peptide oder Peptidderivate sind insbesondere zur Verpackung von Lebensmitteln geeignet, da sie weder beim Kontakt noch bei der Aufnahme toxisch wirken.
Ein anderer Bestandteil dieser Erfindung ist eine Methode zur Behandlung von Säugetieren, die mit Mikroben (insbesondere Bakterien oder Pilze) infiziert sind, einschließlich der Verabreichung einer effektiven, therapeutisch wirksamen Menge der pharmazeutisch wirksamen erfindungsgemäßen Zusammensetzung.
Der hier verwendete Begriff "therapeutisch wirksame Menge" bezeichnet die Menge eines Therapeutikums, d. h. eines erfindungsgemäßen Peptids, Peptidderivats oder -konjugats, welche die Vermehrung und Kolonienbildung der Bakterien zu reduzieren oder ganz zu verhindern oder einen messbaren therapeutischen bzw. prophylaktischen Erfolg zu erzielen vermag. Der Effekt kann beispielsweise für Biopsien in Kultur, durch Test der bakteriellen Aktivität oder mit einer anderen geeigneten Methode zur Beurteilung des Umfangs und des Grads einer bakteriellen Infektion bestimmt werden. Die genaue effektive Menge für ein Subjekt hängt von dessen Größe und Gesundheitszustand, der Art und dem Ausmaß der Erkrankung und den Therapeutika oder der Kombination mehrerer Therapeutika ab, die zur Behandlung ausgewählt wurden. Insbesondere die erfindungsgemäßen pharmazeutischen Zusammensetzungen können verwendet werden, um bakterielle Infektionen und/oder biologische oder physische Begleiterscheinungen (z.B. Fieber) zu reduzieren oder zu verhindern. Methoden zur Festlegung der Anfangsdosis durch einen Mediziner sind Stand der Technik. Die festgelegten Dosen müssen sicher und erfolgreich sein.
Die Menge eines erfindungsgemäßen Peptids, die für eine antibakteriell effektive Dosis notwendig ist, kann unter Berücksichtigung des Pathogens, das die Infektion auslöst, der Schwere der Infektion, sowie vom Alter, Gewicht, Geschlecht, allgemeiner physischer Kondition usw. des Patienten festgelegt werden. Die notwendige Menge des erfindungsgemäßen Peptids, um effektiv antibakteriell und antimykotisch ohne nennenswerte Nebenwirkungen wirksam zu sein, hängt von der eingesetzten pharmazeutischen Rezeptur und der eventuellen Anwesenheit weiterer Bestandteile wie. Antibiotika, Antimykotika usw. ab. Für die erfindungsgemäßen Einsatzgebiete kann eine effektive Dosis zwischen 0,01 nmol/kg und 50 nmol/kg liegen, vorzugsweise zwischen 0,2 nmol/kg und 10 nmol/kg des Peptids, Peptidderivats oder -konjugats in dem behandelten Individuum.
Anfangsdosen der erfindungsgemäßen Peptide, Peptidomimetika, Multimere, Peptid-Konjugate oder Peptidomimetika-Konjugate können optional durch wiederholte Verabreichung verfolgt werden. Die Häufigkeit der Dosierungen hängt von den oben identifizierten Faktoren ab und beträgt vorzugsweise zwischen ein und sechs Dosen pro Tag über einen Behandlungszeitraum von etwa drei Tagen bis maximal einer Woche.

In einer weiteren Ausführungsform werden die Verbindungen pulmonal in einer bestimmten Menge verabreicht, z.B. durch einen Inhalator, Zerstäuber, Aerosolspray oder einen Inhalator für trockenes Pulver. Geeignete Formulierungen können durch bekannte Methoden und Techniken hergestellt werden. Eine transdermale oder rektale Zufuhr mag in einigen Fällen genau wie die Verabreichung in das Auge angebracht sein.

Es kann vorteilhaft sein, die erfindungsgemäßen Substanzen durch fortgeschrittene Formen der Arzneimittelgabe (advanced drug delivery or targeting methods) effektiver zu verabreichen. So kann, falls der Verdauungstrakt umgangen werden soll, die Darreichungsform eine beliebige Substanz oder Mischung enthalten, die die Bioverfügbarkeit steigert. Dies kann beispielsweise dadurch erreicht werden, dass der Abbau reduziert wird, z.B. durch einen Enzyminhibitor oder ein Antioxidans. Besser ist es, wenn die Bioverfügbarkeit der Verbindung durch eine Zunahme der Permeabilität der Absorptionsbarriere, meist die Schleimhaut, erzielt wird. Substanzen, die das Eindringen erleichtern, können auf verschiedene Arten wirken; einige erhöhen die Fluidität der Schleimhaut, während andere die Zwischenräume zwischen den Schleimhautzellen erweitern. Wiederum andere reduzieren die Viskosität des Schleims auf der Schleimhaut. Zu den bevorzugten Aufnahmebeschleunigern gehören amphiphile Substanzen wie Cholinsäurederivate, Phospholipide, Ethanol, Fettsäuren, Ölsäure, Fettsäurederivate, EDTA, Carbomere, Polycarbophil und Chitosan.

Indikationen, für welche die erfindungsgemäßen modifizierten Peptide, deren Konjugate oder Multimere eingesetzt werden können, sind bakterielle Infektionen mit sowohl Gram-positiven als auch Gram-negativen Bakterien, beispielsweise *Escherichia coli, Enterobacter cloacae, Erwinia amylovora, Klebsiella pneumoniae, Morganella morganii, Pseudomonas aeruginosa, Salmonella typhimurium, Salmonella typhi, Shigella dysenteriae, Yersinia enterocolitica, Acinetobacter calcoaceticus, Acinetobacter baumannii, Agrobacterium tumefaciens, Francisella tularensis, Legionella pneumophila, Pseudomonas syringae, Pseudomonas aeruginosa, Rhizobium meliloti, Haemophilus influenzae* und *Staphylococcus aureus.*

Die Erfindung wird nachfolgend durch folgende Ausführungsbeispiele und Figuren erläutert, ohne die Erfindung auf diese zu beschränken:
- **Fig. 1**: zeigt die Ergebnisse der Substitutionsanalyse von Apidaecin 1b (GNNRPVYIPQPRPPHPRL - SEQ ID Nr. 2) mit den jeweils im Einbuchstabencode angegeben Aminosäuren. Werte unter 1 stehen für die Verbesserung der mikrobiellen Aktivität. Werte über 1 für eine Verschlechterung. Die linke Spalte zeigt die native Sequenz. Die obere Zeile zeigt den Substituenten. Der Wert von 0,55 direkt unter dem C beispielsweise ist ein Maß für die Aktivität der nativen Apidaecinsequenz, die an Position 1 mit Cystein substituiert ist.
- **Fig. 2**: zeigt eine bestimmte Ausführungsform der hier beschriebenen Peptide. Jedes mit X markierte Peptid ist wirkungsvoller als das native Apidaecin und insbesondere gegenüber gram-positiven Bakterien vorteilhaft.

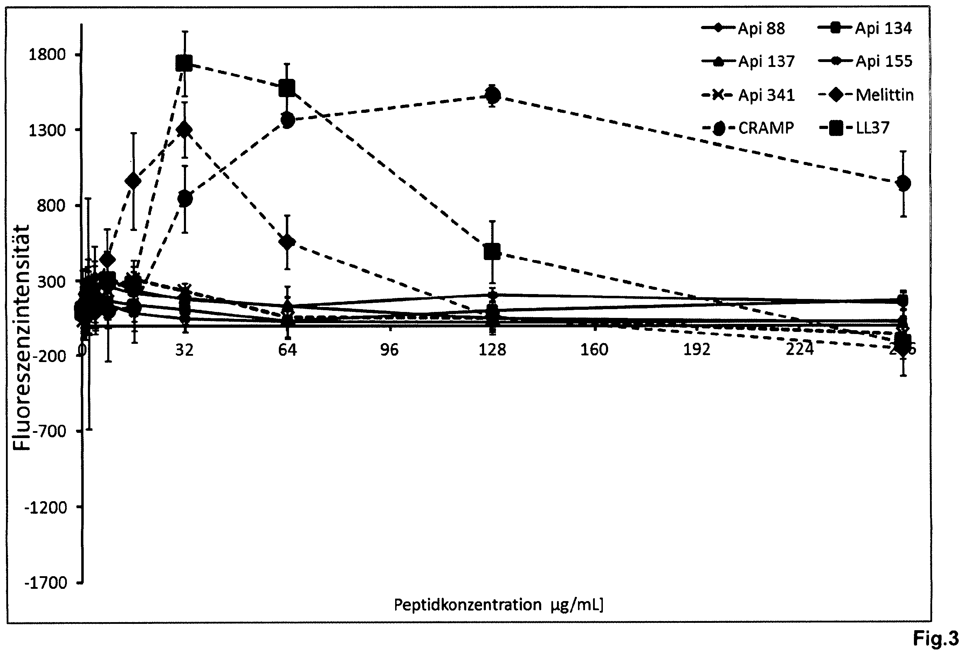

- **Fig. 3**: zeigt eine Permeabilisationsassay. *E. coli* BL21AI mit Apidaecin Derivaten. Api88 entspricht dem Peptid mit SEQ ID NO. 92; Apil37 entspricht dem Peptid mit SEQ ID NO. 93; Api341 entspricht dem Peptid mit SEQ ID NO. 89.

### BEISPIELE

### Beispiel 1: Substitutionsanalyse mittels Peptid-Array

Für die Optimierung der antimikrobiellen Aktivität von Apidaecin 1b wurde mit diesem Peptid (gemäß SEQ ID Nr. 2) eine Substitutionsanalyse durchgeführt. Mittels der SPOT-Synthese wurde die Substitutionsbibliothek synthetisiert und mithilfe des Bioluminenszenz-Assays auf die antibakterielle Aktivität gegenüber *Pseudomonas aeruginosa* untersucht.

Die SPOT-Synthese der Peptidbibliotheken erfolgte auf einem Whatman 50-Filterpapierausschnitt (Sigma-Aldrich, Deutschland) der Größe 19×29 cm mittels Fmoc-Methode und einem SPOT-Synthesizer (Intavis AG, Deutschland) (entsprechend Reineke U et al. 2001) beschrieben. Die verwendete Lumineszenz-Screening-Methode basiert auf der Veröffentlichung von Hilpert und Hancock (Hilpert, 2007. Die auf der Membran synthetisierten Peptide wurden abgespalten und die Peptidspots mit Hilfe eines Lochers aus der Peptidmembran ausgestanzt und in eine 96-Well-Mikrotiterplatte (Corning, USA) überführt und Pro Well 200 µL dest. Wasser zugegeben. Die Platte wurde mit Aluminiumfolie (Biorad, Deutschland) versiegelt und für 18 h bei RT leicht geschüttelt. Auf diese Weise wurde jedes Peptid des Arrays auf genau ein Well einer Mikrotiterplatte übertragen, die Masterplatten genannt werden. Die versiegelten Masterplatten wurden bei -20°C gelagert. Die Masterplatten wurden so konzipiert, dass jede Reihe 10 Peptide sowie zwei Kontrollen (positiv und negativ) enthält.

Im zweiten Schritt erfolgte das eigentliche Screening. Hierfür wurde eine Übernachtkultur (37°C, 225 rpm, 18 h) eines lumineszierenden Bakterienstammes (*P. aeruginosa*) angesetzt. Die Übernachtkultur wurde 100-fach verdünnt und bis zur optischen Dichte von 0,35 bei 600 nm [OD600] wachsen gelassen (ca. 2 Stunden - logarithmische Phase Kultur - LogK). Die Inkubationssuspension (4 vol.% LogK in 100 mM Tris-HCl Puffer (pH 7,3) mit 40 mM sterilfiltrierter Glukose) wurde nun auf lumineszenzgeeignete 96-Well-Platten (VWR, Deutschland) verteilt und mit einer Konzentrationsreihe der Peptidbibliothek 4 h lang bei 37°C inkubiert. Nach der Inkubation erfolgte die Lumineszenz-Messung mithilfe des Luminometers (Thermo, Finnland).

Aus den Ergebnissen der Substitutionsanalyse wurden die Peptidsequenzen ausgewählt, die im Assay die höchste Aktivität zeigten. Diese Peptide wurden konventionell an einem polymeren Träger synthetisiert und auf ihre antibakterielle Aktivität gegen *P. aeruginosa, E.coli* und *S. aureus* mittels des MHK-Assays untersucht.

### Beispiel 2: Bestimmung der minimalen Hemmkonzentrationen und Wachstumskinetik

Die minimalen Hemmkonzentrationen (MHK) der Peptide wurden in einer Doppelbestimmung von Triplikaten mit einer Positiv-(Gentamycin) und einer Negativkontrolle (0,9 % NaCl-Lösung) nach einem modifizierten Protokoll aus Wiegand et al. (Wiegand, 2008) ermittelt.

Die Peptide wurden dazu in Wasser gelöst und in einer zweifachen Verdünnungsreihe mit 1/8 MH (achtfach verdünntes Mueller-Hinton-Medium - 2,6 g/L, Merck) in sterilen 96-Well-Platten (Greiner Bio-One GmbH) von 128 µg/mL in zwölf Verdünnungsschritten auf 62,5 ng/mL verdünnt. Übernachtkulturen wurden mit 1/8 MHB auf ca. 1,5 x 10⁷ Kolonie bildende Einheiten pro mL eingestellt. Davon wurden jeweils 50 µL Peptidlösung pro Well mit je 50 µL der Bakterienlösung vermischt, um eine Anfangskonzentration von 4 x 10⁵ Bakterien pro Well zu erreichen. Nach 20 Stunden Inkubation bei 37 °C wurde die Absorption bei 595 nm (Mikroplatten-Leser, Wallac Victor3, Perkin Elmer) bestimmt. Die minimale Hemmkonzentration wurde als die niedrigste Peptidkonzentration identifiziert, bei der kein bakterielles Wachstum nachgewiesen wurde.

Im Experiment wurde die antibakterielle Wirkung der erfindungsgemäßen Peptide gegenüber den folgenden Bakterienstämmen analysiert: *Pseudomonas aeruginosa PAO1 (wt strain), Pseudomonas aeruginosa* DSM 9644, *Staphylococcus aureus* DSM 1104 /ATCC 25923, *Staphylococcus aureus* ATCC 6247, *Escherichia coli* UB1005 (F-, nalA37, metB1) und *Escherichia coli* ATCC25922. In der folgenden Tabelle 4 sind die Ergebnisse des Versuchs dargestellt:

**Tabelle 4: Minimale Hemmkonzentrationen in 1/8 MHB in µg/mL**

| | | **MHK [µg/mL]** | | | **Verbesserung gegenüber nativer Sequenz (SEQ Nr. 2)** | | |
|---|---|---|---|---|---|---|---|
| SEQ ID Nr. | Peptid | *P. aeruginosa* PAO1 wt | *E. coli* UB1005 | *S*. *aureus* ATCC 25923 | *P. aeruginosa* PAO1 wt | *E. coli* UB1005 | *S. aureus* ATCC 25923 |
| 2* | GNNRPVYIPQPRPPHPRL-**OH** | 500 | 5 | >125 | **1** | **1** | **1** |
| 90* | GNNRPVYIPQPRPPHPRL-NH₂ | 250 | 1,25 | >125 | **2** | **4** | |
| 54 | G**W**WNRPVYIPQPRPPHPRL-NH₂ | 64 | 1,25 | 63 | **8** | **4** | **4** |
| 55 | G**R**NRPVYIPQPRPPHPRL-NH₂ | 64-128 | 0,625 | 32 | **4-8** | **8** | **8** |
| 56 | GNNR**C**VYIPQPRPPHPRL-NH₂ | 125 | 10 | 31 | **4** | **1** | **8** |
| 57 | GNNR**R**VYIPQPRPPHPRL-NH₂ | 64 | 5 | 32 | **8** | **1** | **8** |
| 58 | GNNRPVY**R**PQPRPPHPRL-NH₂ | 64 | 0,313 | 63 | **8** | **16** | **4** |
| 59 | GNNRPVYIPQPR**R**PHPRL-NH₂ | 125 | 10 | 31 | **4** | **1** | **8** |
| 60 | GNNRPVYIPQPRP**C**HPRL-NH₂ | 250 | 20 | **16** | **2** | **0** | **16** |
| 61 | GNNRPVYIPQPRPPH**C**RL-NH₂ | 125 | 20 | 32 | **4** | **0** | **8** |
| 62 | GNNRPVYIPQPRPPHPR**R**-NH₂ | 125 | 1,25-2,5 | 125 | **4** | **2-4** | **2** |
| 63 | G**W**NRPVYIP**R**PRPPHPRL-NH₂ | 16-32 | 0,63 | **16** | **16-32** | **8** | **16** |
| 52 | G**W**NRPVYIPQPR**R**PHPRL-NH₂ | 64 | 5 | 4-8 | **8** | **1** | **31-64** |
| 64 | GNNRPVYIP**R**PR**R**PHPRL-NH₂ | 64 | 2,5 | 4 | **8** | **2** | **64** |
| 50 | G**W**NRPVYIP**R**PR**R**PHPRL-NH₂ | 32 | 2,5 | 2 | **16** | **2** | **128** |
| 91* | GNN**D**PVYIPQPRPPHPRL-NH₂ | 121,0 | >19,4 | >60,5 | **4** | **<0,25** | |

| | | | | | | | |
|---|---|---|---|---|---|---|---|
| * Vergleichsbeispiele | | | | | | | |

Die Ergebnisse zeigen, dass die Modifikationen (insbesondere N2W und/oder P15R) die antimikrobielle Aktivität, insbesondere gegenüber *P. aeruginosa* und *S. aureus* deutlich erhöht.

Zudem wurde die antibakterielle Wirkung der erfindungsgemäßen Peptide gegenüber den folgenden pathogenen Bakterienstämmen des gram-positiven Bakteriums *S. aureus* sowie von P. *aeruginosa* analysiert:

**Tabelle 5: Antimikrobielle Aktivität gegenüber unterschiedlichen pathogenen Stämmen von S. aureus, und P. aeruginosa Stämme. MHK-Werte wurden in 1/8 MHB in µg/mL in Triplikaten bestimmt.**

| SEQ ID Nr. | | *S. aureus* DSM 6247 | *S. aureus* DSM 1104/ATCC 25923 | *E. coli* ATCC 25922 | *P. aeruginosa* PAO DSM 9644 | *P. aeruginosa* PAO1 wt |
|---|---|---|---|---|---|---|
| 2* | GNNRPVYIPQPRPPHPRL-OH | 256 | >125 | 2 | >256 | 500 |
| 90* | GNNRPVYIPQPRPPHPRL-NH₂ | 64 | >125 | 2 | n.b. | 250 |
| 52 | GWNRPVYIPQPRRPHPRL-NH₂ | 2 | 4-8 | 16 | 64 | 64 |
| 64 | GNNRPVYIPRPRRPHPRL-NH₂ | 2 | 4 | 8 | 32 | 64 |
| 50 | GWNRPVYIPRPRRPHPRL-NH₂ | 2 | 2 | 8 | 32 | 32 |

| | | | | | | |
|---|---|---|---|---|---|---|
| * Vergleichsbeispiele, n.b.: nicht bestimmt | | | | | | |

In einem weiteren Experiment wurde die die antibakterielle Wirkung erfindungsgemäßer Peptide mit guanidiertem N-Terminus gegenüber den folgenden pathogenen Bakterienstämmen des gram-positiven Bakteriums *S. aureus* sowie von P. *aeruginosa* analysiert:

**Tabelle 6: Antimikrobielle Aktivität gegenüber unterschiedlichen pathogenen Stämmen von S. aureus, und P. aeruginosa Stämme. MHK-Werte wurden in 1/8 MHB in µg/mL in Triplikaten bestimmt.**

| SEQ ID Nr. | | *S. aureus* DSM 6247 | *P. aeruginosa* PAO DSM 9644 | *E. coli* DSM 1103 |
|---|---|---|---|---|
| 84 | gu-ONNRPVYIPRPRPPHPRR-OH | 8 | 64 | 4 |
| 85 | gu-OWNRPVYIPRPRPPHPRL-OH | 16 | 16 | 4 |
| 86 | gu-ONNRPVYIPRPRRPHPRL-OH | 32 | 32 | 8 |
| 87 | gu-OWNRPVYIPRPRRPHPRL-OH | 16 | 8 | 8 |
| 88 | gu-ONNRPVYIPRPRRPHPRL-NH₂ | 16 | 16 | 8 |
| 89 | gu-OWNRPVYIPRPRRPHPRL-NH₂ | 8-16 | 4 | 8 |

| | | | | |
|---|---|---|---|---|
| *Gu: N-Terminus enthält eine Tetramethylguanidinogruppe (N-guanido-Ornithin)* | | | | |

Weitere Messungen ergaben die folgenden Ergebnisse.

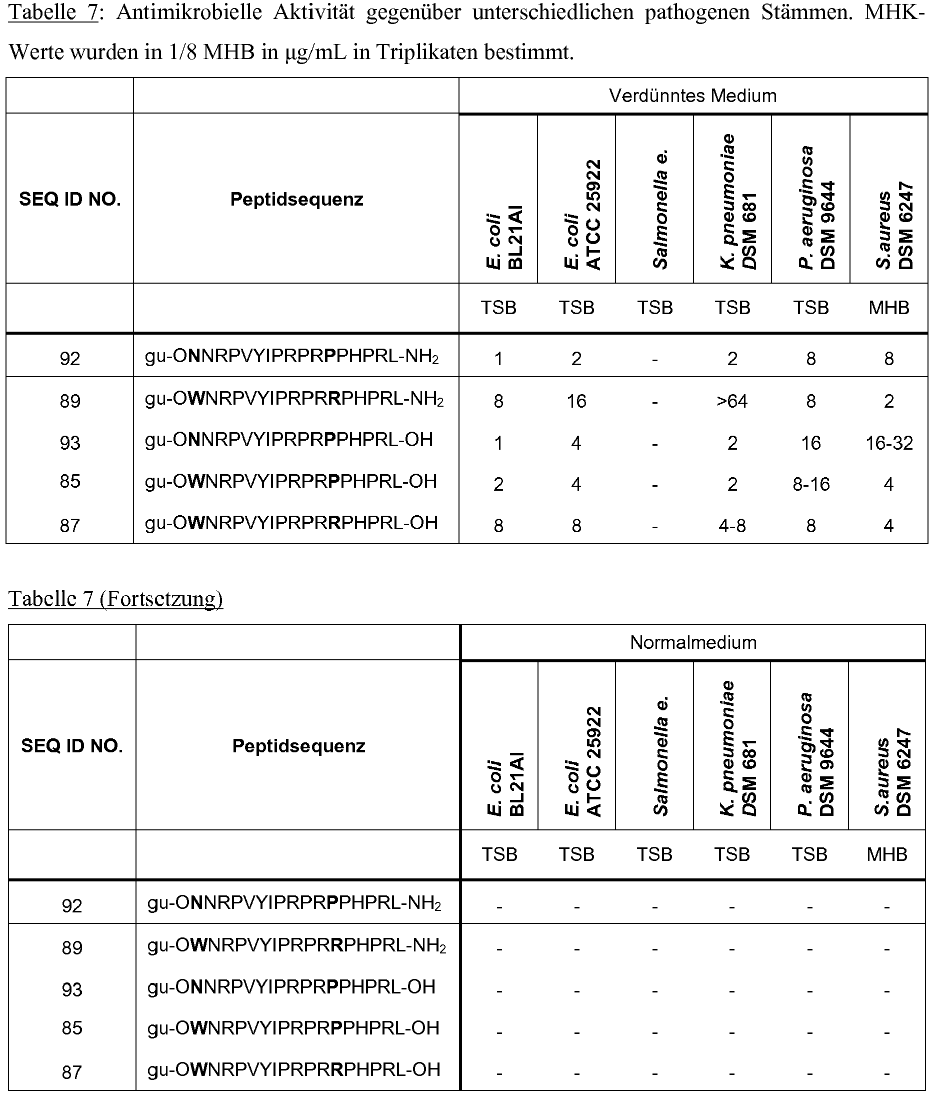

Desweiteren wurde die Serumstabilität ausgewählter Apidaecinderivate gemessen. Die Ergebnisse sind in Tabelle 8 dargestellt.

**Tabelle 8. Serumstabilität der Apidaecin WR Derivate in 25% und 100% Mausserum.**

| **SEQ ID NO.** | **Sequenz^{a}** | **t½ [min] 25% serum** | **t½ [min] 100% serum** | **Abbauprodukte** |
|---|---|---|---|---|
| 92 | gu-O**N**NRPVYIPRPR**P**PHPRL-NH₂ | 15 ± 1 | | gu-O1-R17 |
| 89 | gu-O**W**NRPVYIPRPR**R**PHPRL-NH₂ | 16 ± 1 | | gu-O1-R17, gu-O1-R12 |
| 93 | gu-O**N**NRPVYIPRPR**P**PHPRL-OH | | 350 ± 34 | gu-O1-R17 |
| 85 | gu-O**W**NRPVYIPR**P**RPPHPRL-OH | | 237±15 | gu-O1-R17, gu-O1-R16 |
| 87 | gu-O**W**NRPVYIPRPRRPHPRL-OH | | 0% nach 2h | gu-O1-R12 (analog Api341) |

### In der Erfindungsbeschreibung werden folgende Abkürzungen verwendet:

- BOC: *tert*.-Butyloxy-carbonyl
- ^{t}Bu: *tert*.-Butylether
- DCM: Dichlormethan
- DMF: Dimethylformamid
- *eq.*: *equivalents per mol,* Moläquivalente
- Fmoc: Fluorenylmethoxycarbonyl
- Guan: Guanidino-Gruppe (am N-Terminus)
- Hyp: *trans*-4-Hydroxyprolin
- HBTU: 2-(1H-Benzotriazol-1-yl)-1,1,3,3-tetramethyluronium-hexafluorophosphat
- HOBt: 1-Hydroxybenzotriazol
- M: mol/l
- MALDI-TOF: Matrix unterstützte Laser-Desorption/Ionisierung (engl. *Matrix Assisted Laser Desorption*/*Ionization)* mit Flugzeitanalyse (engl. *time of flight*)
- MHK: minimalen Hemmkonzentration
- MS: Massenspektrometrie (engl. *Mass Spectrometry*)
- Mtt: 4-Methyltrityl
- NHS: *N*-Hydroxysuccinimid
- NMM: *N*-Methylmorpholine
- O: Ornithin
- O^{t}Bu: *tert*.-Butylester
- PBS: Phosphat-gepufferte Salzlösung (engl. *Phospho-buffered Saline*)
- RP-HPLC: Umkehrphasen-Hochleistungsflüssigkeitschromatographie (engl. *reversed phase high performance liquid chromatography*)
- RT: Raumtemperatur
- TCA: Trichloressigsäure
- TFA: Trifluoressigsäure
- Tris: Tris(hydroxymethyl)-aminomethan
- TSB: *Tryptic Soy Broth,* tryptisches Soja Medium

### Zitierte Nichtpatentliteratur

Barra, D., Simmaco, M., and Boman, H.G. (1998) Gene encoded peptide antibiotics and innate immunity. Do 'animacules' have defense budgets? FEBS Lett. 430: 130-134.
Boman, H.G. (1995) Peptide antibiotics and their role in innate immunity. Annu. Rev. Immunol. 13: 61-92.
Czihal P. et al. (2007) Int J Antimicrob Agents 29, S. 602
Ellman, J., Mendel, D., Anthony-Cahill, S., Noren, C.J., Schultz, P.G. (1991) Biosynthetic method for introducing unnatural amino acids site-specifically into proteins. Meth. Enzymol. 202: 301-336.
Gobbo, M., Biondi, L., Filira, F. und Rocchi R. (2006) The interaction of cationic antimicrobial peptides with vesicles containing synthetic glycolipids as models of the outer membrane of gram-negative bacteria. J. Pept. Sci. 12: 132-9.
Hilpert K. und Hancock, R.E. (2007) Use of luminescent bacteria for rapid screening and characterization of short cationic antimicrobial peptides synthesized on cellulose using peptide array technology. Nat. Protoc. 2: 1652-60.
Li, W.F., Ma, G.X. und Zhou, X.X. (2006) Apidaecin-type peptides: biodiversity, structurefunction relationships and mode of action. Peptides. 27: 2350-9.
Maeno, M., Taguchi, S. und Momose, H. (1993) Production of antibacterial peptide 'apidaecin' using the secretory expression system of Streptomyces. Biosci. Biotechnol. Biochem. 57: 1206-7.
Noren, C.J., Anthony-Cahill, S.J., Griffith, M.C. and Schultz, P.G. (1989) A general method for site-specific incorporation of unnatural amino acids into proteins. Science 244: 182-188.
Otvos, L., Jr., Bokonyi, K., Varga, I., Otvos, B.I., Hoffmann, R., Ertl, H.C.J., Wade, J.D., McManus, A.M., Craik, D.J. und Bulet, P. (2000) Insect peptides with improved proteaseresistance protect mice against bacterial infection. Protein Sci. 9: 742-749.
Reineke, U., Volkmer-Engert, R. und Schneider-Mergener, J. (2001) Applications of peptide arrays prepared by the SPOT-technology. Curr. Opin. Biotechnol. 12: 59-64.
Wiegand, I., Hilpert, K. und Hancock, R.E.(2008) Agar and broth dilution methods to determine the minimal inhibitory concentration (MIC) of antimicrobial substances. Nat. Protoc. 3: 163-75.

## Patentansprüche

1. Ein Peptid zur Verwendung als Medikament in der Behandlung einer Infektion mit Gram positiven Bakterien enthaltend eine Aminosäuresequenz gemäß der allgemeinen Formel A oder B:
X₁-N₂-X₂-X₃-P₅-V₆-Y₇-I₈-P₉-X₄-X₅-R₁₂-P₁₃-P₁₄-H₁₅-P₁₆ (Formel A)
X₁-N₂-X₂-X₃-P₅-V₆-Y₇-I₈-P₉-X₄-X₅-R₁₂-P₁₃-P₁₄-H₁₅-P₁₆-X₆-X₇ (Formel B)
wobei die Aminosäuresequenz gemäß Formel A oder B mindestens 80 % Aminosäuresequenzidentität zu nativem Apidaecin Ib gemäß SEQ ID Nr. 2 aufweist und wobei gilt:
X₁ ist ausgewählt aus unpolaren, aromatischen, positiv geladenen Aminosäureresten, Aminosäureresten mit einer Thiol-Gruppe, Aminosäureresten mit einer Selenol-Gruppe, Prolin und Prolinderivaten;
N₂, X₂ und P₅ sind unabhängig voneinander ausgewählt aus neutralen und positiv geladenen Aminosäureresten;
X₃ ist ausgewählt aus positiv geladenen Aminosäureresten, Aminosäureresten mit einer Thiol- Gruppe und Aminosäureresten mit einer Selenol-Gruppe;
V₆ ist ausgewählt aus unpolaren Aminosäureresten mit mindestens 2 C-Atomen in der Seitenkette, aromatischen Aminosäureresten, positiv geladenen Aminosäureresten, Aminosäureresten mit einer Thiol-Gruppe, Aminosäureresten mit einer Selenol-Gruppe, Prolin und Prolinderivaten;
Y₇ ist ausgewählt aus Tyrosin, positiv geladenen Aminosäureresten, Aminosäureresten mit einer Thiol-Gruppe und Aminosäureresten mit einer Selenol-Gruppe;
I₈ ist ausgewählt aus unpolaren, aromatischen Aminosäureresten mit mindestens 2 und maximal 8 C-Atomen in der Seitenkette, positiv geladenen Aminosäureresten, Aminosäureresten mit einer Thiol-Gruppe und Aminosäureresten mit einer Selenol-Gruppe;
P₉, P₁₃, P₁₄ und P₁₆ sind unabhängig voneinander ausgewählt aus positiv geladenen Aminosäureresten, Aminosäureresten mit einer Thiol-Gruppe, Aminosäureresten mit einer Selenol-Gruppe, unpolaren aromatischen Aminosäureresten, heteroaromatischen Aminosäureresten, Prolin und Prolinderivaten;
X₄ ist ausgewählt aus neutralen, positiv geladenen Aminosäureresten, Aminosäureresten mit einer Thiol-Gruppe und Aminosäureresten mit einer Selenol-Gruppe;
X₅ ist ausgewählt aus Prolin, Prolinderivaten, positiv geladenen Aminosäureresten, Aminosäureresten mit einer Thiol-Gruppe und Aminosäureresten mit einer Selenol-Gruppe;
R₁₂ ist ein positiv geladener Aminosäurerest;
H₁₅ ist ausgewählt aus Histidin, positiv geladenen Aminosäureresten, Aminosäureresten mit einer Thiol-Gruppe und Aminosäureresten mit einer Selenol-Gruppe;
X₆ ist ausgewählt ist aus positiv geladenen Aminosäureresten,
X₇ ist ausgewählt aus unpolaren Aminosäureresten, positiv geladenen Aminosäureresten, Aminosäureresten mit einer Thiol-Gruppe und Aminosäureresten mit einer Selenol-Gruppe,
**dadurch gekennzeichnet dass** zumindest eine der Positionen 2, 5 bis 11, 13 bis 16 und 18 von SEQ ID No. 2 so verändert ist, dass auf das Peptid gemäß Formel A oder B mindestens eine der folgenden Bedingungen zutrifft:
N₂ ist Tryptophan oder Arginin, und/oder
I₈ ist Arginin.

2. Das Peptid gemäß Anspruch 1 enthaltend eine Aminosäuresequenz gemäß einer der SEQ ID, 50, 52 und 54 bis 55, 58 und 63.

3. Das Peptid gemäß einem der Ansprüche 1 bis 2, wobei die N-terminale Aminosäure und/oder die C-terminale Aminosäure modifiziert sind, vorzugsweise wobei die N-terminale Aminosäure guanidiert ist.

4. Das Peptid gemäß einem der Ansprüche 1 bis 2, in welchem der N-Terminus und/oder der C- Terminus direkt oder über einen Linker mit mindestens einem weiteren Peptid, Protein, Polymer und/oder Träger verbunden ist.

5. Das Peptid gemäß einem der Ansprüche 1 bis 3, **dadurch gekennzeichnet, dass** mindestens eine der Peptidbindungen des Peptidrückgrats chemisch modifiziert ist.

6. Ein Peptid enthaltend eine Aminosäuresequenz gemäß den Ansprüchen 1 bis 4.

7. Eine pharmazeutische Zusammensetzung, **dadurch gekennzeichnet, dass** sie mindestens ein Peptid gemäß Anspruch 5 enthält.

8. Eine Nukleinsäure codierend für ein Peptid gemäß Anspruch 5.

9. Eine Wirtszelle, welche eine Nukleinsäure gemäß Anspruch 7 enthält.

10. Ein Peptid-Multimer, umfassend mindestens zwei Peptide nach Anspruch 5, wobei die mindestens zwei Peptide miteinander über ein Linkerpeptid verknüpft sind.

## Claims

1. A peptide for use as a medicament in the treatment of infection with gram-positive bacteria containing an amino acid sequence according to the general formula A or B:
X₁-N₂-X₂-X₃-P₅-V₆-Y₇-I₈-P₉-X₄-X₅-R₁₂-P₁₃-P₁₄-H₁₅-P₁₆ (formula A)
X₁-N₂-X₂-X₃-P₅-V₆-Y₇-I₈-P₉-X₄-X₅-R₁₂-P₁₃-P₁₄-H₁₅-P₁₆-X₆-X₇ (formula B)
wherein the amino acid sequence according to formula A or B has at least 80% amino acid sequence identity to native apidaecin lb according to SEQ ID No. 2 and in which:
X₁ is selected from nonpolar, aromatic, positively charged amino acid residues, amino acid residues with a thiol group, amino acid residues with a selenol group, proline and proline derivatives;
N₂, X₂ and P₅ are independently selected from neutral and positively charged amino acid residues;
X₃ is selected from positively charged amino acid residues, amino acid residues having a thiol group, and amino acid residues having a selenol group;
V₆ is selected from nonpolar amino acid residues having at least 2 C-atoms in the side chain, aromatic amino acid residues, positively charged amino acid residues, amino acid residues having a thiol group, amino acid residues having a selenol group, proline and proline derivatives;
Y₇ is selected from tyrosine, positively charged amino acid residues, amino acid residues having a thiol group, and amino acid residues having a selenol group;
I₈ is selected from nonpolar, aromatic amino acid residues having at least 2 and a maximum of 8 C-atoms in the side chain, positively charged amino acid residues, amino acid residues having a thiol group and amino acid residues having a selenol group;
P₉, P₁₃, P₁₄ and P₁₆ are independently selected from positively charged amino acid residues, amino acid residues having a thiol group, amino acid residues having a selenol group, nonpolar aromatic amino acid residues, heteroaromatic amino acid residues, proline and proline derivatives;
X₄ is selected from neutral, positively charged amino acid residues, amino acid residues having a thiol group, and amino acid residues having a selenol group;
X₅ is selected from proline, proline derivatives, positively charged amino acid residues, amino acid residues having a thiol group, and amino acid residues having a selenol group;
R₁₂ is a positively charged amino acid residue;
H₁₅ is selected from histidine, positively charged amino acid residues, amino acid residues having a thiol group and amino acid residues having a selenol group;
X₆ is selected from positively charged amino acid residues,
X₇ is selected from nonpolar amino acid residues, positively charged amino acid residues, amino acid residues having a thiol group and amino acid residues having a selenol group,
**characterised in that** at least one of the positions 2, 5 to 11, 13 to 16 and 18 of SEQ ID no. 2 is modified so that the peptide according to formula A or B has at least one of the following conditions:
N₂ is tryptophan or arginine, and/or
I₈ is arginine.

2. The peptide according to claim 1 containing an amino acid sequence according to one of the SEQ ID, 50, 52 and 54 to 55, 58 and 63.

3. The peptide according to any one of claims 1 to 2, wherein the N-terminal amino acid and/or the C-terminal amino acid are modified, preferably wherein the N-terminal amino acid is guanidinated.

4. The peptide according to any one of claims 1 to 2, in which the N-terminus and / or the C-terminus are linked directly or via a linker to at least another peptide, protein, polymer and/or support.

5. The peptide according to any one of claims 1 to 3, **characterized in that** at least one of the peptide bonds of the peptide backbone is chemically modified.

6. A peptide containing an amino acid sequence according to claims 1 to 4.

7. A pharmaceutical composition **characterized in that** it contains at least one peptide according to claim 5.

8. A nucleic acid encoding a peptide according to claim 5.

9. A host cell containing a nucleic acid according to claim 7.

10. A peptide multimer comprising at least two peptides according to claim 5, wherein the at least two peptides are linked via a linker peptide.

## Revendications

1. Peptide à utiliser comme médicament dans le traitement d'infection par des bactéries à Gram positif contenant une séquence d'acides aminés selon la formule générale A ou B:
X₁-N₂-X₂-X₃-P₅-V₆-Y₇-I₈-P₉-X₄-X₅-R₁₂-P₁₃-P₁₄-H₁₅-P₁₆ (formule A)
X₁-N₂-X₂-X₃-P₅-V₆-Y₇-I₈-P₉-X₄-X₅-R₁₂-P₁₃-P₁₄-H₁₅-P₁₆-X₆-X₇ (formule B)
dans lequel la séquence d'acides aminés selon la formule A ou B a au moins 80% d'identité de séquence d'acides aminés avec l'apidaécine Ib native conformément à la SEQ ID No 2 et dans laquelle:
X₁ est choisi parmi des résidus d'acides aminés non polaires, aromatiques, chargés positivement, des résidus d'acides aminés ayant un groupe thiol, des résidus d'acides aminés ayant un groupe sélénol, proline et dérivés de la proline;
N₂, X₂ et P₅ sont indépendamment choisis parmi des résidus d'acides aminés neutres et des résidus d'acides aminés chargés positivement;
X₃ est choisi parmi des résidus d'acides aminés chargés positivement, des résidus d'acides aminés ayant un groupe thiol et des résidus d'acides aminés ayant un groupe sélénol;
V₆ est choisi parmi des résidus d'acides aminés non polaires ayant au moins 2 atomes de carbone dans la chaîne latérale, des résidus d'acides aminés aromatiques, des résidus d'acides aminés chargés positivement, des résidus d'acides aminés ayant un groupe thiol, des résidus d'acides aminés ayant un groupe sélénol, la proline et des dérivés de la proline;
Y₇ est choisi parmi la tyrosine, des résidus d'acides aminés chargés positivement, des résidus d'acides aminés ayant un groupe thiol et des résidus d'acides aminés ayant un groupe sélénol;
I₈ est choisi parmi des résidus d'acides aminés non polaires, des résidus d'acides aminés aromatiques ayant au moins 2 et un maximum de 8 atomes de carbone dans la chaîne latérale, des résidus d'acides aminés chargés positivement, des résidus d'acides aminés ayant un groupe thiol et des résidus d'acides aminés ayant un groupe sélénol;
P₉, P₁₃, P₁₄ et P₁₆ sont indépendamment choisis parmi des résidus d'acides aminés chargés positivement, des résidus d'acides aminés ayant un groupe thiol, des résidus d'aminoacides ayant un groupe sélénol, des résidus d'acides aminés aromatiques non polaires, des résidus d'acides aminés hétéroaromatiques, la proline et des dérivés de la proline;
X₄ est choisi parmi des résidus d'acides aminés neutres, des résidus d'acides aminés chargés positivement, des résidus d'acides aminés ayant un groupe thiol et des résidus d'acides aminés ayant un groupe sélénol;
X₅ est choisi parmi la proline, des dérivés de la proline, des résidus d'acides aminés chargés positivement, des résidus d'acides aminés ayant un groupe thiol et des résidus d'acides aminés ayant un groupe sélénol;
R₁₂ est un résidu d'acides aminés chargé positivement;
H₁₅ est choisi parmi l'histidine, des résidus d'acides aminés chargés positivement, des résidus d'acides aminés ayant un groupe thiol et des résidus d'acides aminés ayant un groupe sélénol;
X₆ est choisi parmi des résidus d'acides aminés chargés positivement,
X₇ est choisi parmi des résidus d'acides aminés non polaires, des résidus d'acides aminés chargés positivement, des résidus d'acides aminés ayant un groupe thiol et des résidus d'acides aminés ayant un groupe sélénol,
**caractérisé en ce qu'au** moins une des positions 2, 5 à 11, 13 à 16 et 18 de la SEQ ID no. 2 est modifiée pour que le peptide de formule A ou B ait au moins une des conditions suivantes:
N₂ est le tryptophane ou l'arginine et/ou
I₈ est l'arginine.

2. Peptide selon la revendication 1 ayant une séquence d'acides aminés selon l'une des SEQ ID, 50, 52 et 54 à 55, 58 et 63.

3. Peptide selon l'une quelconque des revendications 1 à 2, dans lequel l'acide aminé N-terminal et/ou l'acide aminé C-terminal sont modifiés, de préférence dans lequel l'acide aminé N-terminal est guanidiné.

4. Peptide selon l'une quelconque des revendications 1 à 2, dans lequel l'extrémité N-terminale et/ou l'extrémité C-terminale sont liées directement ou via un lieur, à au moins un autre peptide, une protéine, un polymère et/ou un support.

5. Peptide selon l'une quelconque des revendications 1 à 3, **caractérisé en ce qu'**au moins l'une des liaisons peptidiques du squelette peptidique est modifiée chimiquement.

6. Peptide contenant une séquence d'acides aminés selon les revendications 1 à 4.

7. Composition pharmaceutique, **caractérisée en ce qu'**elle contient au moins un peptide selon la revendication 5.

8. Acide nucléique codant pour un peptide selon la revendication 5.

9. Cellule hôte contenant un acide nucléique selon la revendication 7.

10. Multimère peptidique comprenant au moins deux peptides selon la revendication 5, dans lequel les au moins deux peptides sont liés par l'intermédiaire d'un peptide de liaison.
